# FASCICULE DE BREVET EUROPEEN

(11) **EP 3 178 381 B1**
(45) Date de publication et mention de la délivrance du brevet: **20.03.2019**
(21) Numéro de dépôt: 16202680.1
(22) Date de dépôt: 07.12.2016
(51) Int. Cl.: A61B 5/00, A61B 5/11

(54) **DISPOSITIF ET PROCÉDÉ D'ÉVALUATION POUR ÉVALUER DES PARAMÈTRES MORPHOLOGIQUES D'UN MEMBRE INFERIEUR**
VORRICHTUNG UND VERFAHREN ZUR AUSWERTUNG DER MORPHOLGISCHEN PARAMETER EINES UNTEREN GLIEDS
EVALUATION DEVICE AND METHOD FOR EVALUATING MORPHOLOGICAL PARAMETERS OF A LOWER LIMB

(30) Priorité: 08.12.2015 FR 1562007
(43) Date de publication de la demande: 14.06.2017
(73) Titulaire: Amplitude, 26000 Valence (FR)
(72) Inventeur: HULET, Christophe, 14000 Caen (FR); POTEL, Jean-François, 31500 Toulouse (FR); ROCHCONGAR, Goulven, 14610 Cambes en Plaine (FR); COLOMBET, Philippe, 33700 Mérignac (FR); GRAVELEAU, Nicolas, 33000 Bordeaux (FR); SEIL, Romain, 5441 Schengen (LU)
(74) Mandataire: Chevalier, Renaud Philippe

(56) Documents cités:
- US-A1- 2010 125 229
- TURCOT K ET AL: "New Accelerometric Method to Discriminate Between Asymptomatic Subjects and Patients With Medial Knee Osteoarthritis During 3-D Gait", IEEE TRANSACTIONS ON BIOMEDICAL ENGINEERING, IEEE SERVICE CENTER, PISCATAWAY, NJ, USA, vol. 55, no. 4, 1 avril 2008 (2008-04-01), pages 1415-1422, XP011342670, ISSN: 0018-9294, DOI: 10.1109/TBME.2007.912428
- SHAFAGH GANJIKIA ET AL: "Three-dimensional knee analyzer validation by simple fluoroscopic study", THE KNEE, vol. 7, no. 4, 1 décembre 2000 (2000-12-01), pages 221-231, XP055292258, AMSTERDAM, NL ISSN: 0968-0160, DOI: 10.1016/S0968-0160(00)00063-6

## Description

La présente invention concerne un dispositif d'évaluation pour évaluer des paramètres morphologiques d'un membre inférieur d'un patient, en particulier pendant une consultation préopératoire ou postopératoire, et concerne également un procédé d'évaluation mettant en oeuvre un tel dispositif d'évaluation.

La présente invention s'applique notamment au domaine de la chirurgie orthopédique ou ligamentaire du genou. En particulier, la présente invention peut s'appliquer à une opération de pose d'une prothèse de genou, partielle ou totale, ou à une opération de reconstruction ou de remplacement ligamentaire, par exemple pour le genou.

FR3009677A1 décrit un dispositif d'évaluation de paramètres morphologiques d'un membre inférieur. Ce dispositif d'évaluation comprend des accéléromètres fémoraux et des accéléromètres tibiaux qui sont solidarisés directement à la cuisse et à la jambe par des sangles. Ce dispositif d'évaluation comprend en outre une unité de calcul pour recevoir les signaux générés par les accéléromètres fémoraux et tibiaux et pour calculer la position relative du fémur par rapport au tibia.

Cependant, un tel dispositif d'évaluation de l'état de la technique n'a pas une précision suffisante pour déterminer d'autres paramètres morphométriques du membre inférieur, notamment les centres de la tête fémorale, du genou et de la cheville. D'une part, les accéléromètres n'offrent pas une précision de positionnement suffisante pour déterminer exactement leurs positions. D'autre part, chaque accéléromètre est solidarisé individuellement et localement sur la cuisse ou la jambe. Or, l'élasticité des muscles et de la peau induit des déplacements parasites des accéléromètres, ce qui diminue la précision des évaluations effectuées.

L'état de la technique peut également être illustré par l'enseignement donné dans l'article « New accelerometric method to discriminate between asymptomatic subjects and patients with medial knee osteoarthritis during 3D gait », TURCOT K et al., IEEE Transactions on Biomedical Engineering, IEEE Service Center, Piscataway, NJ, USA, vol.55, no. 4, 2008-04-01, pages 1415-1422, XP011342670, ISSN : 0018-9294, DOI : 10.1109/TBME.2007.912428. Cet article divulgue un dispositif d'évaluation pour évaluer des paramètres morphologiques d'un membre inférieur d'un patient, comprenant un exosquelette pourvu d'un ensemble fémoral comprenant un gyromètre fémoral triaxial et un accéléromètre fémoral triaxial, et un ensemble tibial comprenant un gyromètre tibial triaxial et un accéléromètre tibial triaxial. Ce dispositif d'évaluation comprend un jeu de six caméras optoélectroniques propres à filmer et suivre des marqueurs réflectifs placés sur l'exosquelette. Des marqueurs sont également placés sur la malléole médiale et sur la malléole latérale, afin que les caméras permettent d'établir la position du centre de la cheville durant les manipulations.

Le dispositif d'évaluation présenté dans cet article est ainsi un dispositf très complexe et coûteux car, en plus de l'exosquelette équipé de gyromètres et d'accéléromètres, il est également prévu un jeu de six caméras. Le premier inconvénient d'un tel dispositif d'évaluation est qu'il nécessite un traitement de trop nombreuses données, à savoir les signaux issus des gyromètres et des accéléromètres, mais également des signaux vidéo issus des six caméras qui appelent un traitement d'image particlièrement lourd et donc coûteux en ressources et en temps de traitement. Le second inconvénient d'un tel dispositif d'évaluation est qu'il fait appel à trop de matériel, en particulier les caméras qui doivent être positionnés de manière précise, ce qui contribue à le rendre inaccessible pour certains praticiens, tant en termes de coût que d'utilisation.

La présente invention a notamment pour but de résoudre, en tout ou partie, les problèmes mentionnés ci-avant.

Dans ce but, la présente invention a pour objet un dispositif d'évaluation, pour évaluer des paramètres morphologiques d'un membre inférieur d'un patient, en particulier pendant une consultation préopératoire ou postopératoire, le dispositif d'évaluation étant caractérisé en ce qu'il comprend au moins :
- un ensemble fémoral de capteurs inertiels comprenant trois gyromètres fémoraux et trois accéléromètres fémoraux, les gyromètres fémoraux étant configurés pour générer respectivement des signaux représentatifs de leurs vitesses angulaires respectives autour des trois dimensions d'un référentiel galiléen, les accéléromètres fémoraux étant configurés pour générer des signaux représentatifs de leurs accélérations linéaires respectives selon les trois dimensions du référentiel galiléen,
- un ensemble tibial de capteurs inertiels comprenant trois gyromètres tibiaux et trois accéléromètres tibiaux, les gyromètres tibiaux étant configurés pour générer respectivement des signaux représentatifs de leurs vitesses angulaires respectives autour des trois dimensions d'un référentiel galiléen, les accéléromètres tibiaux étant configurés pour générer des signaux représentatifs de leurs accélérations linéaires respectives selon les trois dimensions du référentiel galiléen,
- un support fémoral configuré pour maintenir l'ensemble fémoral de capteurs inertiels sur la cuisse du patient, le support fémoral ayant : i) une portion proximale de fixation fémorale configurée pour fixer le support fémoral sur une portion proximale de la cuisse, et ii) une portion distale de fixation fémorale configurée pour fixer le support fémoral sur une portion distale de la cuisse,
- un support tibial configuré pour maintenir l'ensemble tibial de capteurs inertiels sur la jambe du patient, le support tibial ayant : i) une portion proximale de fixation tibiale configurée pour fixer le support tibial sur une portion proximale de la jambe et ii) une portion distale de fixation tibiale configurée pour fixer le support tibial sur une portion distale de la jambe,
- un palpeur malléolaire fixé au support tibial, le palpeur malléolaire comprenant une branche médiale et une branche latérale qui sont articulées au support tibial de façon à être déplaçables entre :
   i) une configuration de contact, dans laquelle une zone de contact de la branche médiale et une zone de contact de la branche latérale sont mises en contact respectivement avec la malléole médiale et avec la malléole latérale, et
   ii) une configuration distante, dans laquelle la branche médiale et la branche latérale sont distantes respectivement de la malléole médiale et de la malléole latérale, et
- une unité de calcul configurée pour recevoir les signaux générés par les gyromètres fémoraux, par les accéléromètres fémoraux, par les gyromètres tibiaux et par les accéléromètres tibiaux, l'unité de calcul étant en outre configurée pour :
   - recevoir lesdits signaux générés lorsque l'ensemble tibial de capteurs inertiels suit un premier mouvement circulaire induit lorsque le membre inférieur en extension ou en hyperextension tourne autour de la tête fémorale du patient,
   - calculer, à partir des signaux reçus, la position du centre de la tête fémorale,
   - recevoir lesdits signaux générés lorsque l'ensemble fémoral de capteurs inertiels est immobile, la cuisse du patient étant en immobile et en extension ou en hyperextension, et lorsque l'ensemble tibial de capteurs inertiels suit un deuxième mouvement circulaire induit par au moins une flexion de la jambe supérieure à 60 degrés, par exemple supérieure à 90 degrés,
   - calculer, à partir des signaux reçus, la position du centre du genou,
   - recevoir lesdits signaux générés lorsque la branche médiale et la branche latérale sont mises en contact respectivement avec la malléole médiale et avec la malléole latérale pendant que le pied est immobile, et
   - calculer, à partir desdits signaux reçus, la position du centre de la cheville comme étant le milieu du segment délimité par la zone de contact de la branche médiale et la zone de contact de la branche latérale lorsque la branche médiale et la branche latérale sont en configuration de contact.

Ainsi, l'évaluation de ces paramètres morphologiques permet à un praticien, lors d'une consultation préopératoire ou postopératoire, de modéliser la morphométrie du patient, ce qui facilite l'opération chirurgicale. En particulier, modéliser la morphométrie du patient permet au praticien d'évaluer la flexion du genou et/ou d'évaluer la laxité des ligaments du membre inférieur, en particulier du genou, en varus, en valgus et/ou en rotation interne/ rotation externe. En effet, lorsque l'unité de calcul a évalué les positions respectives du centre de la tête fémorale, du centre du genou et du centre de la cheville, l'unité de calcul peut déterminer : i) un axe mécanique fémoral reliant le centre de la tête fémorale au centre du genou et ii) un axe mécanique tibial reliant le centre du genou au centre de la cheville. L'axe mécanique fémoral et l'axe mécanique tibial forment trois angles dans l'espace :
i) un angle dans le plan frontal qui correspond au varus ou valgus du membre inférieur ;
ii) un angle dans le plan horizontal qui correspond à la rotation interne ou externe ;
iii) un angle dans le plan sagittal est la flexion.

Les laxités de ligaments sont les amplitudes de ces trois angles.

Lorsque le dispositif d'évaluation est en service, pendant le premier mouvement circulaire, l'ensemble fémoral de capteurs inertiels suit un mouvement circulaire identique au mouvement suivi par l'ensemble tibial de capteurs inertiels, car le membre inférieur est verrouillé en hyperextension ou en extension (rectiligne). Lorsque le dispositif d'évaluation est en service, pendant le premier mouvement circulaire, le bassin du patient est immobile. Le centre de la tête fémorale correspond au centre de l'articulation de la hanche.

Lorsque le dispositif d'évaluation est en service, pendant le deuxième mouvement circulaire, la cuisse est en position d'extension.

En outre, un tel dispositif d'évaluation ne nécessite pas de système de prise d'image, tel qu'une caméra. Le dispositif d'évaluation selon l'invention est utilisable directement, sans avoir à placer précisément un ou des systèmes de prise d'image autour du patient, et fait appel à des ressources réduites car aucun traitement d'image ne sera mis en oeuvre par ce dispositif d'évaluation.

Selon une variante, le centre de la cheville est défini comme le milieu du segment formé par les points palpés correspondant respectivement à la malléole médiale et à la malléole latérale.

Selon une variante, l'unité de calcul peut comprendre au moins un microcontrôleur, par exemple du modèle PIC32 commercialisé par la société *Microchip Technology.*

Selon une variante, la flexion de la jambe couvre un angle de flexion supérieur à 90 degrés à partir de la jambe en extension.

Selon une variante, l'unité de calcul est en outre configurée pour calculer la position du centre du genou à partir des signaux générés lorsque l'ensemble tibial de capteurs inertiels suit un deuxième mouvement circulaire induit successivement par un mouvement composé d'une flexion de la jambe supérieure à 60 degrés, et d'une extension ramenant la jambe en position d'extension.

En particulier, l'unité de calcul est en outre configurée pour calculer la position du centre du genou à partir des signaux générés lorsque l'ensemble tibial de capteurs inertiels suit un deuxième mouvement circulaire induit successivement par plusieurs flexions de la jambe supérieures à 60 degrés et par plusieurs extensions ramenant la jambe en position d'extension.

Selon une variante, l'ensemble fémoral de capteurs inertiels comprend un boîtier fémoral configuré pour loger les gyromètres fémoraux et accéléromètres fémoraux. Ainsi, un tel boîtier fémoral protège les gyromètres fémoraux et les accéléromètres fémoraux. En particulier, chacune des dimensions du boîtier fémoral peut être inférieure à 100 mm, voire inférieure à 60 mm. Ainsi, un tel boîtier fémoral tient dans une main, ce qui facilite sa manipulation.

Selon une variante, l'ensemble tibial de capteurs inertiels comprend un boîtier tibial configuré pour loger les gyromètres tibiaux et accéléromètres tibiaux. Ainsi, un tel boîtier tibial protège les gyromètres fémoraux et les accéléromètres fémoraux. En particulier, chacune des dimensions du boîtier tibial peut être inférieure à 100 mm, voire inférieure à 60 mm. Ainsi, un tel boîtier tibial tient dans une main, ce qui facilite sa manipulation.

Selon une variante, le dispositif d'évaluation comprend plusieurs ensembles fémoraux de capteurs inertiels. Selon une variante, le dispositif d'évaluation comprend plusieurs ensembles tibiaux de capteurs inertiels.

Selon une variante, l'un au moins parmi le boîtier fémoral et le boîtier tibial comprend un élément de détrompage. Ainsi, un utilisateur peut assembler sans erreur le boîtier fémoral sur le support fémoral et le boîtier tibial sur le support tibial.

Par exemple, chaque accéléromètre fémoral et chaque accéléromètre tibial peut être du modèle ADXL330® commercialisé par la société *Analog Devices.* Par exemple, chaque gyromètre fémoral et chaque gyromètre tibial peut être du modèle L3G4200D® commercialisé par la société *STMicroelectronics.* Dans la présente demande, le terme « gyromètre » désigne tout appareil configuré pour générer un signal représentatif d'une vitesse angulaire, d'une accélération angulaire ou d'une position angulaire, tel qu'un gyroscope.

Lorsque le dispositif d'évaluation est en service, après la palpation des malléoles médiale et latérale, le support peut être mis dans une position de référence, qui est identifiable à tout moment au cours du procédé d'évaluation. Ainsi, le dispositif de mesure peut retrouver cette position de référence en cas de dérive des accélérations et des intégrations.

Selon une variante, les gyromètres fémoraux, les accéléromètres fémoraux, les gyromètres tibiaux et les accéléromètres tibiaux sont configurés pour générer lesdits signaux lorsque l'ensemble tibial de capteurs inertiels suit : i) un mouvement de flexion sur une plage angulaire de -25 degrés à +165 degrés, ii) un mouvement de varus ou de valgus sur une plage angulaire de -30 degrés à +30 degrés, et un iii) un mouvement de rotation interne/externe du genou sur une plage angulaire de -45 degrés et +45 degrés.

Selon une variante, le palpeur malléolaire est fixé au support tibial de manière amovible, par exemple par des composants élastiquement encliquetables.

Selon une variante, la branche médiale et la branche latérale sont articulées au support tibial selon une liaison pivot. Selon une variante, la branche médiale et la branche latérale peuvent présenter un mécanisme d'entraînement configuré de sorte que la branche médiale et la branche latérale suivent des déplacements simultanés et d'amplitudes limitées, ce qui améliore la précision de l'évaluation. En d'autres termes, un déplacement de la branche médiale entraîne un déplacement de la branche latérale et réciproquement. Par exemple, la branche médiale et la branche latérale peuvent comporter des dents d'entraînement qui sont complémentaires et qui sont agencées pour s'engrener mutuellement de façon à déplacer simultanément la branche médiale et la branche latérale, donc à limiter la liberté et l'amplitude de déplacement d'une branche par rapport à l'autre.

Selon un mode de réalisation, le support fémoral comprend un élément fémoral de liaison reliant mécaniquement la portion proximale de fixation fémorale à la portion distale de fixation fémorale, l'élément fémoral de liaison étant rigide, l'ensemble fémoral de capteurs inertiels étant solidarisé à l'élément fémoral de liaison.

Ainsi, l'ensemble fémoral de capteurs inertiels est lié rigidement à l'élément fémoral de liaison.

Dans la présente demande, le terme « rigide » qualifie un élément dont la raideur est supérieure à la raideur d'un os, par exemple à la raideur du fémur. Par exemple, un os cortical peut avoir un module de Young compris entre 6 et 20 GPa, alors qu'un élément fémoral de liaison en titane a un module de Young environ égal à 115 GPa.

Selon une variante, l'ensemble fémoral de capteurs inertiels est solidarisé directement à l'élément fémoral de liaison. Alternativement à cette variante, l'ensemble fémoral de capteurs inertiels peut être solidarisé indirectement à l'élément fémoral de liaison.

Selon un mode de réalisation, l'élément fémoral de liaison comprend une barre fémorale s'étendant de la portion proximale de fixation fémorale à la portion distale de fixation fémorale, la barre fémorale ayant : i) une partie proximale rectiligne et destinée à s'étendre le long de la cuisse, et ii) une partie distale curviligne et configurée pour le passage de plusieurs doigts entre la cuisse et la partie distale.

Ainsi, une telle barre fémorale permet de construire un élément fémoral de liaison simple et peu encombrant. La partie distale de forme curviligne, qui pointe vers l'extérieur, permet au praticien de manipuler la cuisse sans toucher, donc sans déplacer, le support fémoral.

Selon un mode de réalisation, le support tibial comprend un élément tibial de liaison reliant mécaniquement la portion proximale de fixation tibiale à la portion distale de fixation tibiale, l'élément tibial de liaison étant rigide, l'ensemble tibial de capteurs inertiels étant solidarisé à l'élément tibial de liaison.

Ainsi, l'ensemble tibial de capteurs inertiels est lié rigidement à l'élément tibial de liaison rigide.

Selon une variante, l'ensemble tibial de capteurs inertiels est solidarisé directement à l'élément tibial de liaison rigide. Alternativement à cette variante, l'ensemble tibial de capteurs inertiels peut être solidarisé indirectement à l'élément tibial de liaison rigide.

Selon un mode de réalisation, l'élément tibial de liaison comprend une barre tibiale s'étendant de la portion proximale de fixation tibiale à la portion distale de fixation tibiale, la barre tibiale ayant : i) une partie proximale rectiligne et destinée à s'étendre le long de la cuisse, et ii) une partie distale curviligne et configurée pour le passage de plusieurs doigts entre la jambe et la partie distale.

Ainsi, une telle barre tibiale permet de construire un élément tibial de liaison simple et peu encombrant. La partie distale de forme curviligne, qui pointe vers l'extérieur, permet au praticien de manipuler la jambe sans toucher, donc sans déplacer, le support fémoral.

Selon un mode de réalisation, la portion proximale de fixation fémorale a une surface proximale de contact qui est incurvée et destinée à s'appuyer contre une surface proximale de la cuisse, et la portion distale de fixation fémorale a une surface distale de contact qui est incurvée et destinée à s'appuyer contre une surface distale de la cuisse.

Ainsi, de telles surfaces proximale et distale de contact incurvées permettent des appuis sur d'assez grandes surfaces de la cuisse, ce qui répartit la charge.

En pratique, la portion proximale de fixation fémorale et la portion distale de fixation fémorale sont fixées sur la face antérieure de la cuisse lorsque le patient est allongé sur le dos.

Selon un mode de réalisation, le support tibial comprend une partie tibiale télescopique agencée entre la portion proximale de fixation tibiale et la portion distale de fixation tibiale, la partie tibiale télescopique étant configurée pour régler la longueur du support tibial.

Ainsi, une telle partie tibiale télescopique permet d'adapter la longueur du support tibial à la morphologie du patient.

Selon une variante, le support fémoral comprend une partie fémorale télescopique qui est agencée entre la portion proximale de fixation fémorale et la portion distale de fixation fémorale configurée, de façon à régler la longueur du support fémoral. Ainsi, une telle partie fémorale télescopique permet d'adapter la longueur du support fémoral à la morphologie du patient.

Selon un mode de réalisation, la portion proximale de fixation fémorale comprend au moins une sangle fémorale proximale qui est configurée pour entourer la portion proximale de la cuisse, et la portion distale de fixation fémorale comprend au moins une sangle fémorale distale qui est configurée pour entourer la portion distale de la cuisse,

et la portion proximale de fixation tibiale comprend au moins une sangle tibiale proximale qui est configurée pour entourer la portion proximale de la jambe, et la portion distale de fixation tibiale comprend au moins une sangle tibiale distale qui est configurée pour entourer la portion distale de la jambe,
la portion proximale de fixation tibiale comprenant en outre : i) un organe de serrage proximal configuré pour serrer simultanément les deux brins de la sangle tibiale proximale, et ii) un organe de serrage distal configuré pour serrer simultanément les deux brins de la sangle tibiale distale.

Ainsi, de tels organes de serrage proximal et distal permettent de serrer rapidement chaque sangle tibiale sans couple de rotation autour de l'axe mécanique tibial, ce qui garantit un positionnement précis du support tibial sur la jambe.

Selon un mode de réalisation, le dispositif d'évaluation comprend en outre une liaison filaire reliant électriquement l'ensemble fémoral de capteurs inertiels et l'ensemble tibial de capteurs inertiels.

Ainsi, une telle liaison filaire permet de transmettre des signaux et/ou un courant de puissance entre l'ensemble tibial de capteurs inertiels, l'ensemble fémoral de capteurs inertiels et l'unité de calcul.

Selon un mode de réalisation, l'ensemble fémoral de capteurs inertiels est équipé d'une batterie configurée pour alimenter électriquement l'ensemble fémoral de capteurs inertiels et l'ensemble tibial de capteurs inertiels.

Alternativement à ce mode de réalisation, l'ensemble tibial de capteurs inertiels peut être équipé d'une batterie spécifique et indépendante de l'ensemble fémoral de capteurs inertiels.

Selon un mode de réalisation, l'unité de calcul est configurée pour calculer, à partir des signaux reçus, la position du centre de la tête fémorale comme étant une approximation par la méthode des moindres carrés du centre de rotation autour duquel tourne l'ensemble tibial de capteurs inertiels lorsque l'ensemble tibial de capteurs inertiels suit le premier mouvement circulaire.

Ainsi, une telle unité de calcul permet de déterminer précisément la position du centre de la tête fémorale.

Selon un mode de réalisation, l'unité de calcul est configurée pour calculer, à partir des signaux reçus, la position du centre du genou comme étant une approximation par la méthode des moindres carrés du centre de rotation autour duquel tourne l'ensemble tibial de capteurs inertiels lorsque l'ensemble tibial de capteurs inertiels suit le deuxième mouvement circulaire.

Ainsi, une telle unité de calcul permet de déterminer précisément la position du centre du genou.

Selon un mode de réalisation, l'unité de calcul est en outre configurée pour déterminer la position d'un axe mécanique fémoral et la position d'un axe mécanique tibial, l'unité de calcul est en outre configurée pour calculer un angle cuisse-jambe s'étendant, du côté médial, entre l'axe mécanique fémoral et l'axe mécanique tibial.

Ainsi, une telle unité de calcul permet de déterminer précisément les positions de l'axe mécanique tibial et de l'axe mécanique fémoral.

Selon une variante, l'unité de calcul est en outre configurée pour calculer, à partir desdits signaux générés : i) un axe médiolatéral tibial correspondant à la direction passant par la malléole médiale et par la malléole latérale ; et ii) un axe médiolatéral fémoral porté par un vecteur moyen de rotation du repère tibial par rapport au repère fémoral, sur une plage angulaire de flexion s'étendant de 40 degrés à 80 degrés.

Selon une variante, le dispositif d'évaluation comprend en outre une mémoire incluant un modèle anatomique d'un membre inférieur, le modèle anatomique incluant : i) un centre de la tête fémorale, ii) un centre du genou, iii) un centre de la cheville, iv) un axe mécanique fémoral passant par le centre de la tête fémorale et par le centre du genou, et v) un axe mécanique tibial passant par le centre du genou et par le centre de la cheville.

Selon un mode de réalisation, l'ensemble fémoral de capteurs inertiels comprend en outre une interface fémorale de commande tactile, et l'ensemble tibial de capteurs inertiels comprend en outre une interface tibiale de commande tactile.

Selon un mode de réalisation, le dispositif d'évaluation comprend en outre :
- une interface de commande configurée pour permettre à un utilisateur de commander l'unité de calcul, l'interface de commande comprenant un module d'affichage, et
- au moins un module de communication sans fil configuré pour communiquer les résultats desdits calculs à l'interface de commande.

Les modes de réalisation et les variantes mentionnés ci-avant peuvent être pris isolément ou selon toute combinaison techniquement possible.

L'invention se rapporte également à un procédé d'évaluation pour évaluer des paramètres morphologiques d'un membre inférieur d'un patient, en particulier pendant une consultation préopératoire ou postopératoire, ledit procédé d'évaluation mettant en oeuvre les étapes suivantes :
- mettre en oeuvre un dispositif d'évaluation selon l'invention sur ledit membre inférieur ;
- lorsque le dispositif d'évaluation est en service, mettre en marche le dispositif d'évaluation de façon à alimenter électriquement l'ensemble fémoral de capteurs inertiels et l'ensemble tibial de capteurs inertiels ;
- manipuler le membre inférieur pour que l'unité de calcul reçoive les signaux générés par les gyromètres fémoraux, par les accéléromètres fémoraux, par les gyromètres tibiaux et par les accéléromètres tibiaux, de sorte que l'unité de calcul :
   - reçoive lesdits signaux générés lorsque l'ensemble tibial de capteurs inertiels suit un premier mouvement circulaire induit lorsque le membre inférieur en extension ou en hyperextension tourne autour de la tête fémorale du patient,
   - calcule, à partir des signaux reçus, la position du centre de la tête fémorale,
   - reçoive lesdits signaux générés lorsque l'ensemble fémoral de capteurs inertiels est immobile, la cuisse du patient étant en immobile et en extension ou en hyperextension, et lorsque l'ensemble tibial de capteurs inertiels suit un deuxième mouvement circulaire induit par au moins une flexion de la jambe supérieure à 60 degrés, par exemple supérieure à 90 degrés,
   - calcule, à partir des signaux reçus, la position du centre du genou,
   - reçoive lesdits signaux générés lorsque la branche médiale et la branche latérale sont mises en contact respectivement avec la malléole médiale et avec la malléole latérale pendant que le pied est immobile, et
   - calcule, à partir desdits signaux reçus, la position du centre de la cheville comme étant le milieu du segment délimité par la zone de contact de la branche médiale et la zone de contact de la branche latérale lorsque la branche médiale et la branche latérale sont en configuration de contact.

La présente invention sera bien comprise et ses avantages ressortiront aussi à la lumière de la description qui va suivre, donnée uniquement à titre d'exemple non limitatif et faite en référence aux figures schématiques annexées, dans lesquelles des signes de références identiques correspondent à des éléments structurellement et/ou fonctionnellement identiques ou similaires. Dans les figures schématiques annexées :
- la figure 1 est une vue illustrant une partie d'un dispositif d'évaluation conforme à l'invention installé sur un membre inférieur d'un patient ;
- la figure 2 est une vue similaire à la figure 1 dans une autre position du membre inférieur (le patient est allongé, mais le le membre inférieur est représenté verticalement pour utiliser au mieux l'espace de la planche) ;
- la figure 3 est une vue du fémur et du tibia du membre inférieur lorsque la jambe est en extension ;
- la figure 4 est une vue en perspective d'un support fémoral appartenant au dispositif d'évaluation de la figure 1 ;
- la figure 5 est une vue en perspective, suivant un angle différent de la figure 4, du support fémoral de la figure 4 ;
- la figure 6 est une vue d'un ensemble fémoral de capteurs inertiels appartenant au dispositif d'évaluation de la figure 1 ;
- la figure 7 est une vue en perspective d'un support tibial appartenant au dispositif d'évaluation de la figure 1 ;
- la figure 8 est une vue en perspective d'une partie du support tibial de la figure 7 ;
- la figure 9 est une vue en perspective, suivant un angle différent de la figure 8, du support tibial de la figure 8 ;
- la figure 10 est une vue en perspective d'un palpeur malléolaire, en configuration de contact, appartenant au support tibial de la figure 7 ;
- la figure 11 est une vue de dessus du palpeur malléolaire de la figure 10, en position distante ;
- la figure 12 est une vue de dessus du palpeur malléolaire de la figure 10, en configuration de contact ;
- la figure 13 est une vue d'une interface de commande appartenant au dispositif d'évaluation de la figure 1 ;
- la figure 14 est une vue illustrant un premier mouvement circulaire suivi par l'ensemble tibial de capteurs inertiels ;
- la figure 15 est une vue illustrant un deuxième mouvement circulaire suivi par l'ensemble tibial de capteurs inertiels ;
- la figure 16 illustre une palpation de la malléole médiale par le palpeur malléolaire des figures 10 à 12 ;
- la figure 17 illustre une palpation de la malléole latérale par le palpeur malléolaire des figures 10 à 12 ; et
- la figure 18 illustre un procédé d'évaluation mettant en oeuvre le dispositif d'évaluation de la figure 1.

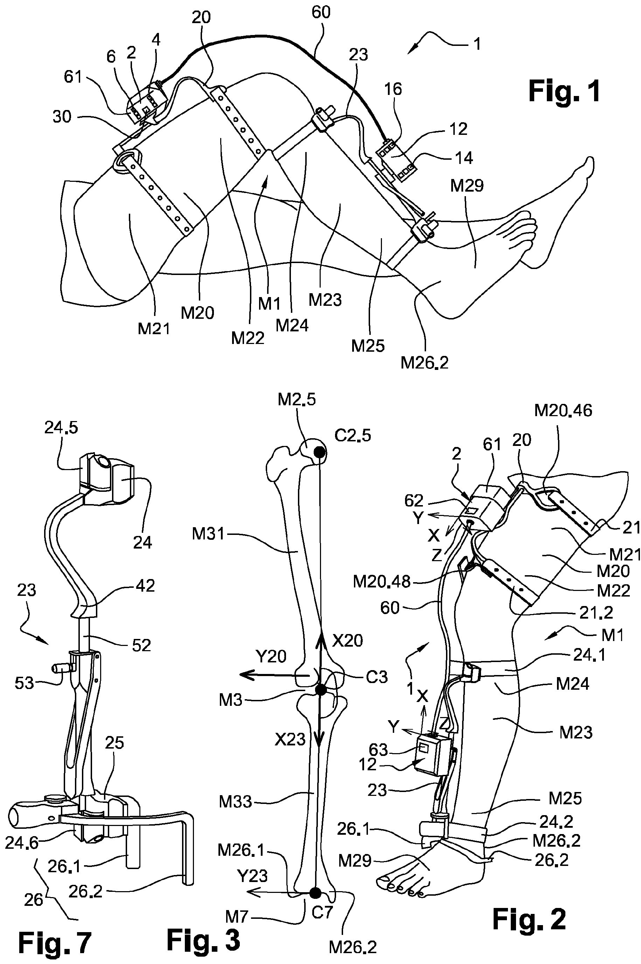

Les figures 1 et 2 illustrent un dispositif d'évaluation 1 pour évaluer des paramètres morphologiques d'un membre inférieur M1 d'un patient. En particulier, le dispositif d'évaluation 1 est destiné à être utilisé pendant une consultation préopératoire ou postopératoire.

Comme le montrent les figures 1 à 3, le membre inférieur M1 comprend une cuisse M20, une jambe M23, un pied M29, un fémur M31, un tibia M33, un genou M3 et une cheville M7.

Le dispositif d'évaluation comprend un ensemble fémoral de capteurs inertiels 2, un ensemble tibial de capteurs inertiels 12, un support fémoral 20, un support tibial 23, un palpeur malléolaire 26, une unité de calcul 30.

L'ensemble fémoral de capteurs inertiels 2 comprend trois gyromètres fémoraux 4 et trois accéléromètres fémoraux 6, qui sont symbolisés à la figure 1. Les gyromètres fémoraux 4 sont ici du modèle L3G4200D® commercialisé par la société *STMicroelectronics.* Les accéléromètres fémoraux 6 sont ici du modèle ADXL330® commercialisé par la société Analog Devices.

Les gyromètres fémoraux 4 sont configurés pour générer respectivement des signaux représentatifs de leurs vitesses angulaires respectives autour des trois dimensions X, Y, Z d'un référentiel galiléen. En d'autres termes, chaque gyromètre fémoral 4 est configuré pour générer des signaux représentatifs de sa vitesse angulaire autour d'une dimension respective X, Y ou Z d'un référentiel galiléen local, qui est ici défini par rapport au fémur M31.

Les accéléromètres fémoraux 6 sont configurés pour générer des signaux représentatifs de leurs accélérations linéaires respectives autour des trois dimensions X, Y, Z du référentiel galiléen. En d'autres termes, chaque accéléromètre fémoral 6 est configuré pour générer des signaux représentatifs de sa vitesse angulaire autour d'une dimension respective X, Y ou Z du référentiel galiléen fémoral.

L'ensemble tibial de capteurs inertiels 12 comprend trois gyromètres tibiaux 14 et trois accéléromètres tibiaux 16, qui sont symbolisés à la figure 1. Les gyromètres tibiaux 14 sont ici du modèle L3G4200D® commercialisé par la société *STMicroelectronics.* Les accéléromètres tibiaux 16 sont ici du modèle ADXL330® commercialisé par la société Analog Devices.

Les gyromètres tibiaux 14 sont configurés pour générer respectivement des signaux représentatifs de leurs vitesses angulaires respectives autour des trois dimensions X, Y, Z d'un référentiel galiléen. En d'autres termes, chaque gyromètre tibial 14 est configuré pour générer des signaux représentatifs de sa vitesse angulaire autour d'une dimension respective X, Y ou Z du référentiel galiléen local, qui est ici défini par rapport au tibia M33.

Les accéléromètres tibiaux 16 sont configurés pour générer des signaux représentatifs de leurs accélérations linéaires respectives autour des trois dimensions X, Y, Z du référentiel galiléen local. En d'autres termes, chaque accéléromètre tibial 16 est configuré pour générer des signaux représentatifs de sa vitesse angulaire autour d'une dimension respective X, Y ou Z du référentiel galiléen tibial.

En outre, le dispositif d'évaluation 1 comprend une liaison filaire 60 reliant électriquement l'ensemble fémoral de capteurs inertiels 2 et l'ensemble tibial de capteurs inertiels 12.

L'ensemble fémoral de capteurs inertiels 2 est ici équipé d'une batterie 61 configurée pour alimenter électriquement l'ensemble fémoral de capteurs inertiels 2 et l'ensemble tibial de capteurs inertiels 12 à travers la liaison filaire 60.

Le support fémoral 20 est configuré pour maintenir l'ensemble fémoral de capteurs inertiels 2 sur la cuisse M20 du patient. Le support fémoral 20 a : i) une portion proximale de fixation fémorale 21 et ii) une portion distale de fixation fémorale 22. La portion proximale de fixation fémorale 21 est configurée pour fixer le support fémoral 20 sur une portion proximale M21 de la cuisse M20. La portion distale de fixation fémorale 22 est configurée pour fixer le support fémoral 20 sur une portion distale M22 de la cuisse M20.

Le support fémoral 20 comprend un élément fémoral de liaison 40 qui relie mécaniquement la portion proximale de fixation fémorale 21 à la portion distale de fixation fémorale 22. L'élément fémoral de liaison 40 est rigide. L'élément fémoral de liaison 40 est ici composé de titane, avec un module de Young environ égal à 115 GPa, alors qu'un os cortical a un module de Young compris entre 6 et 20 GPa.

L'ensemble fémoral de capteurs inertiels 2 est ici solidarisé directement à l'élément fémoral de liaison 40. L'ensemble fémoral de capteurs inertiels 2 est ici solidarisé de manière amovible à l'élément fémoral de liaison 40 par des composants complémentaires élastiquement encliquetables, dont un ergot fémoral 51.

L'ensemble fémoral de capteurs inertiels 2 comprend ici un boîtier fémoral qui est configuré pour loger les gyromètres fémoraux et accéléromètres fémoraux. Chacune des dimensions du boîtier fémoral est ici environ égale à 60 mm.

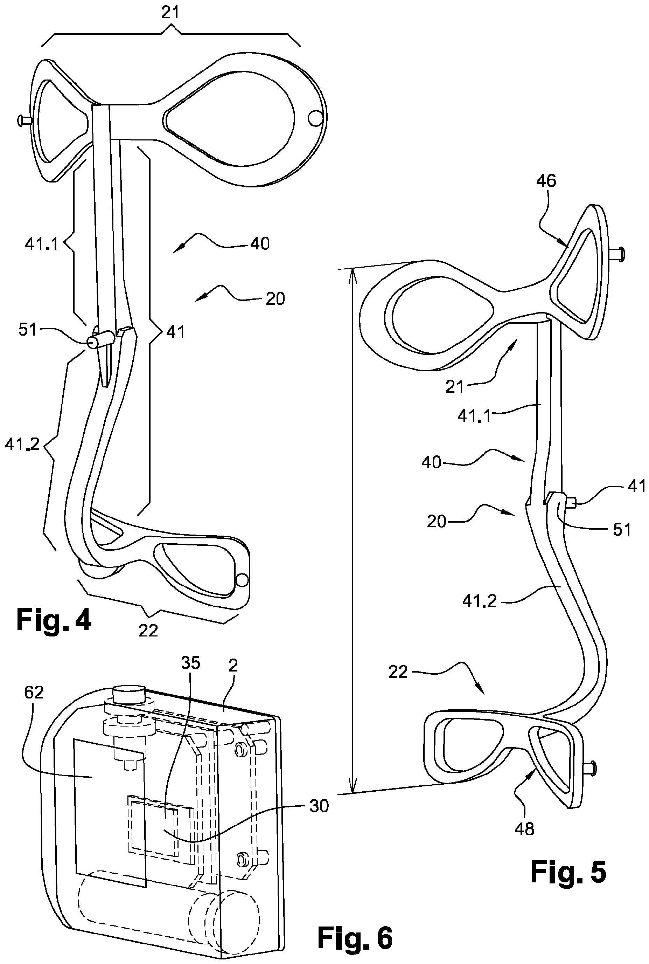

Comme le montrent les figures 4 et 5, l'élément fémoral de liaison 40 comprend une barre fémorale 41 qui s'étend de la portion proximale de fixation fémorale 21 à la portion distale de fixation fémorale 22. La barre fémorale 41 a : i) une partie proximale 41.1 et ii) une partie distale 41.2. La partie proximale 41.1 de la barre fémorale 41 a globalement une forme rectiligne. La partie distale 41.2 de la barre fémorale 41 a globalement une forme curviligne et pointant vers l'extérieur.

Lorsque le dispositif d'évaluation 1 est en service, la partie proximale 41.1 de la barre fémorale 41 s'étend le long de la cuisse M20, alors que la partie distale 41.2 de la barre fémorale 41 permet le passage de plusieurs doigts sous la partie distale 41.2 de la barre fémorale 41 de façon à manipuler la cuisse M20 sans bouger le support fémoral 20.

La portion proximale de fixation fémorale 21 a une surface proximale de contact 46 qui est incurvée. Les courbures de la surface proximale de contact 46 permettent d'appuyer la portion proximale de fixation fémorale 21 contre une surface proximale de la cuisse M20.46.

De même, la portion distale de fixation fémorale 22 a une surface distale de contact 48 qui est incurvée. Les courbures de la surface distale de contact 48 permettent d'appuyer la portion distale de fixation fémorale 22 contre une surface distale de la cuisse M20.48.

Lorsque le dispositif d'évaluation 1 est en service, la portion proximale de fixation fémorale 21 et la portion distale de fixation fémorale 22 sont fixées sur la face antérieure de la cuisse M20 lorsque le patient est allongé sur le dos sur une table de consultation.

Par ailleurs, le support tibial 23 est configuré pour maintenir l'ensemble tibial de capteurs inertiels 12 sur la jambe M23 du patient. Le support tibial 23 a : i une portion proximale de fixation tibiale 24 et ii une portion distale de fixation tibiale 25. La portion proximale de fixation tibiale 24 est configurée pour fixer le support tibial 23 sur une portion proximale M24 de la jambe M23. La portion distale de fixation tibiale 25 est configurée pour fixer le support tibial 23 sur une portion distale M25 de la jambe M23.

Le support tibial 23 comprend un élément tibial de liaison 42 qui relie mécaniquement la portion proximale de fixation tibiale 24 à la portion distale de fixation tibiale 25. L'élément tibial de liaison 42 est rigide. L'élément tibial de liaison 42 est ici composé de titane, avec un module de Young environ égal à 115 GPa, alors qu'un os cortical a un module de Young compris entre 6 et 20 GPa.

L'ensemble tibial de capteurs inertiels 12 est ici solidarisé directement à l'élément tibial de liaison 42. L'ensemble tibial de capteurs inertiels 12 est ici solidarisé de manière amovible à l'élément tibial de liaison 42 par des composants complémentaires élastiquement encliquetables, dont un ergot fémoral 53.

L'ensemble tibial de capteurs inertiels 2 comprend ici un boîtier tibial qui est configuré pour loger les gyromètres tibiaux et accéléromètres tibiaux. Chacune des dimensions du boîtier tibial est ici environ égale à 60 mm.

Comme le montrent les figures 7, 8 et 9, l'élément tibial de liaison 42 comprend une barre tibiale 43 qui s'étend de la portion proximale de fixation tibiale 24 à la portion distale de fixation tibiale 25. La barre tibiale 43 a : i) une partie proximale 43.1 et ii) une partie distale 43.2. La partie proximale 43.1 de la barre tibiale 43 a globalement une forme rectiligne. La partie distale 43.2 de la barre tibiale 43 a globalement une forme curviligne et pointant vers l'extérieur.

Lorsque le dispositif d'évaluation 1 est en service, la partie proximale 43.1 de la barre tibiale 43 s'étend le long de la jambe M23, alors que la partie distale 43.2 de la barre tibiale 43 est configurée pour le passage de plusieurs doigts sous la partie distale 43.2 de façon à manipuler la jambe M23 sans bouger le support tibial 23.

En outre, le support tibial 23 comprend une partie tibiale télescopique 52 qui est agencée entre la portion proximale de fixation tibiale 24 et la portion distale de fixation tibiale 25. La partie tibiale télescopique 52 est configurée pour régler la longueur L23 du support tibial 23, ce qui permet d'adapter la longueur L23 à la morphologie du patient.

De plus, la portion proximale de fixation fémorale 21 comprend au moins une sangle fémorale proximale 21.1 qui est configurée pour entourer la portion proximale M21 de la cuisse M20. La sangle fémorale proximale 21.1 permet d'attacher la portion proximale de fixation fémorale 21 à la cuisse M20.

De même, la portion distale de fixation fémorale 22 comprend une sangle fémorale distale 21.2 qui est configurée pour entourer la portion distale M22 de la cuisse M20. La sangle fémorale distale 21.2 permet d'attacher la portion distale de fixation fémorale 22 à la cuisse M20.

En outre, la portion proximale de fixation tibiale 24 comprend une sangle tibiale proximale 24.1 qui est configurée pour entourer la portion proximale M24 de la jambe M23. La sangle tibiale proximale 24.1 permet d'attacher la portion proximale de fixation tibiale 24 à la jambe M23.

De même, la portion distale de fixation tibiale 25 comprend au moins une sangle tibiale distale 24.2 qui est configurée pour entourer la portion distale M25 de la jambe M23. La sangle tibiale distale 24.2 permet d'attacher la portion distale de fixation tibiale 25 à la jambe M23.

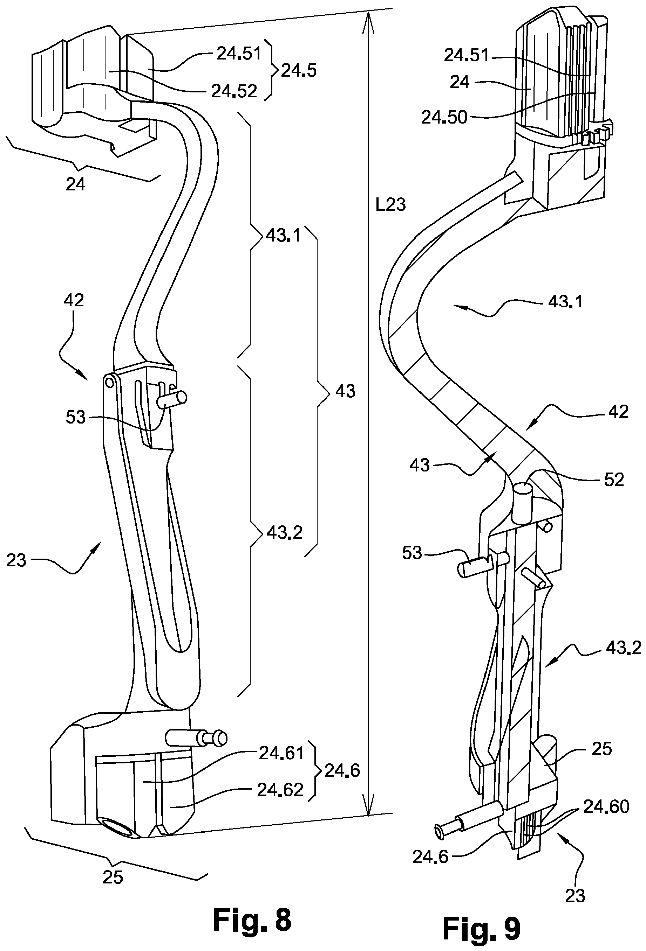

Comme le montrent les figures 8 et 9, la portion proximale de fixation tibiale 24 comprend en outre : i) un organe de serrage proximal 24.5 et ii) un organe de serrage distal 24.6.

L'organe de serrage proximal 24.5 est configuré pour serrer simultanément les deux brins de la sangle tibiale proximale 24.1. À cet effet, l'organe de serrage proximal 24.5 comprend ici deux rouleaux pivotants 24.51 et 24.52. Les rouleaux pivotants 24.51 et 24.52 ont chacun une série de crans 24.50 et équivalent pour serrer simultanément les deux brins de la sangle tibiale proximale 24.1.

L'organe de serrage distal 24.6 est configuré pour serrer simultanément les deux brins de la sangle tibiale distale 24.2. À cet effet, l'organe de serrage distal 24.6 comprend ici deux rouleaux pivotants 24.61 et 24.62. Les rouleaux pivotants 24.61 et 24.62 ont chacun une série de crans 24.60 et équivalent pour serrer simultanément les deux brins de la sangle tibiale distale 24.2.

Le boîtier fémoral de l'ensemble fémoral de capteurs inertiels 12 ou le boîtier tibial de l'ensemble tibial de capteurs inertiels 12 comprend un élément de détrompage, pour que le praticien assemble sans erreur le boîtier fémoral sur le support fémoral 20 et le boîtier tibial sur le support tibial 23.

Comme l'illustrent les figures 7, 10, 11 et 12, le palpeur malléolaire 26 est fixé au support tibial 23, ici par des composants élastiquement encliquetables. Sur la figure 1, le palpeur malléolaire 26 n'apparaît pas, car il est détaché du support tibial 23.

Le palpeur malléolaire 26 comprend une branche médiale 26.1 et une branche latérale 26.2 qui sont articulées au support tibial 23 de façon à être déplaçables entre :
i) une configuration de contact, dans laquelle une zone de contact de la branche médiale 26.1 et une zone de contact de la branche latérale 26.2 sont en contact respectivement avec la malléole médiale M26.1 et avec la malléole latérale M26.2, et
ii) une configuration distante, dans laquelle la branche médiale 26.1 et la branche latérale 26.2 sont distantes respectivement de la malléole médiale M26.1 et de la malléole latérale M26.2.

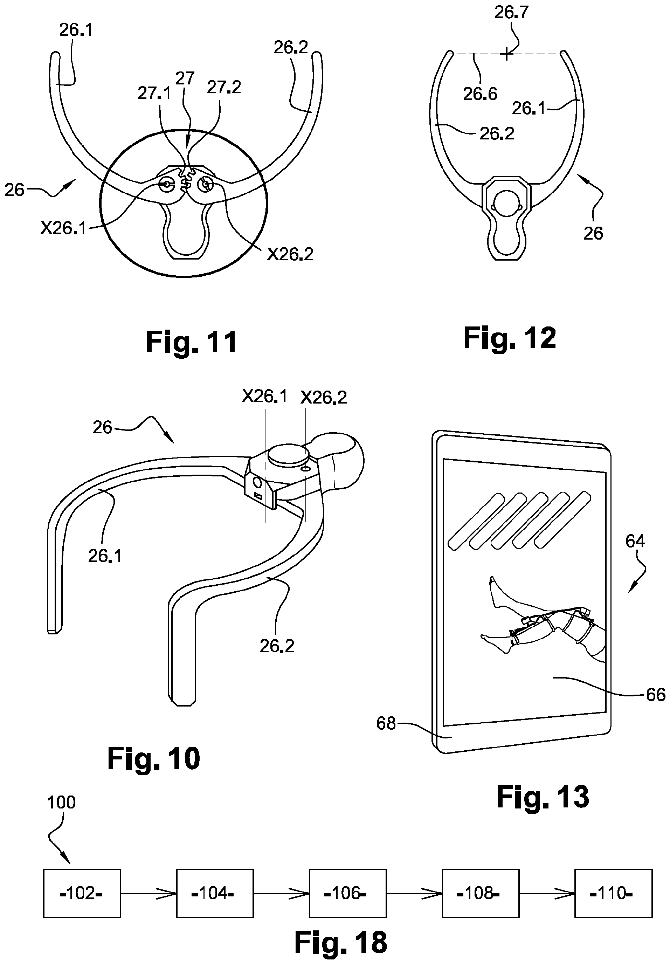

Comme le montre la figure 10, la branche médiale 26.1 comprend une aile intermédiaire en forme de « C » et une portion d'extrémité plate et orthogonale au plan de l'aile intermédiaire. De même, la branche latérale 26.2 comprend une aile intermédiaire en forme de « C » et une portion d'extrémité plate et orthogonale au plan de l'aile intermédiaire.

La branche médiale 26.1 et la branche latérale 26.2 sont articulées au support tibial 23 selon des liaisons pivot respectivement autour d'axes de pivotement X26.1 et X26.2.

La branche médiale 26.1 et la branche latérale 26.2 ont un mécanisme d'entraînement 27 qui est configuré de sorte que la branche médiale 26.1 et la branche latérale 26.2 se déplacent simultanément et avec une amplitude limitée. En d'autres termes, un déplacement de la branche médiale 26.1 entraîne un déplacement de la branche latérale 26.2 et réciproquement. À cet effet, la branche médiale 26.1 et la branche latérale 26.1 comportent chacune des dents d'entraînement 27.1. Les dents d'entraînement 27.1 sont agencées pour s'engrener mutuellement de façon à déplacer la branche médiale 26.1 et la branche latérale 26.2.

Par ailleurs, l'une unité de calcul 30 est configurée pour recevoir les signaux générés par les gyromètres fémoraux 4, par les accéléromètres fémoraux 6, par les gyromètres tibiaux 14 et par les accéléromètres tibiaux 16. L'unité de calcul 30 comprend ici un microcontrôleur du modèle PIC32 commercialisé par la société Microchip Technology.

Lorsque le dispositif d'évaluation est en service, les gyromètres fémoraux 4, les accéléromètres fémoraux 6, les gyromètres tibiaux 14 et les accéléromètres tibiaux 16 génèrent des signaux en permanence, avec un court intervalle de temps entre deux signaux.

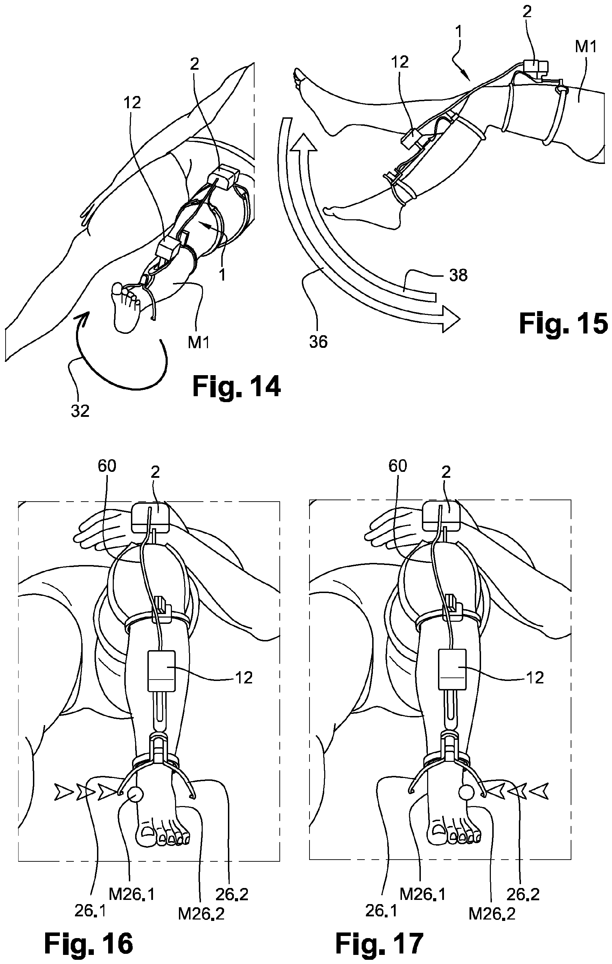

L'unité de calcul 30 est en outre configurée :
- pour recevoir les signaux générés par les gyromètres 4 et accéléromètres 6 (fémoraux) et par les gyromètres 14 et accéléromètres 16 (tibiaux) lorsque l'ensemble tibial de capteurs inertiels 12 suit un premier mouvement circulaire 32, symbolisé à la figure 14, induit lorsque le praticien fait tourner le membre inférieur M1 en extension ou en hyperextension autour de la tête fémorale M2.5 du patient ; et
- pour calculer la position du centre de la tête fémorale C2.5 à partir de ces signaux reçus.

De plus, l'unité de calcul 30 est en outre configurée :
- pour recevoir les signaux générés par les gyromètres 4 et accéléromètres 6 (fémoraux) et par les gyromètres 14 et accéléromètres 16 (tibiaux) lorsque l'ensemble fémoral de capteurs inertiels 2 est immobile, la cuisse M20 du patient étant immobile et en extension ou en hyperextension, et lorsque l'ensemble tibial de capteurs inertiels 12 suit un deuxième mouvement circulaire composé d'une flexion 36 supérieure à 90 degrés, puis d'une extension 38 de la jambe M23 ; le deuxième mouvement circulaire est symbolisé à la figure 15 ; et
- pour calculer la position du centre du genou C3 à partir de ces signaux reçus.

L'unité de calcul 30 est aussi configurée :
- pour recevoir les signaux générés par les gyromètres 4 et accéléromètres 6 (fémoraux) et par les gyromètres 14 et accéléromètres 16 (tibiaux) lorsque la branche médiale 26.1 et la branche latérale 26.2 sont mises en contact respectivement avec la malléole médiale M26.1 et avec la malléole latérale M26.2 pendant que le pied M29 est immobile, et
- pour calculer, à partir de ces signaux reçus, la position du centre de la cheville C7 comme étant le milieu 26.7 du segment 26.6 délimité par la zone de contact (portion d'extrémité) de la branche médiale 26.1 et la zone de contact (portion d'extrémité) de la branche latérale 26.2 lorsque la branche médiale 26.1 et la branche latérale 26.2 sont en configuration de contact (figures 16 et 17) ; en d'autres termes, la position du centre de la cheville C7 comme étant le milieu 26.7 du segment 26.6 formé par les points palpés correspondant respectivement à la malléole médiale M26.1 et à la malléole latérale M26.2.

De plus, l'unité de calcul 30 est configurée pour calculer, à partir des signaux générés par les gyromètres 4 et accéléromètres 6 (fémoraux) et par les gyromètres 14 et accéléromètres 16 (tibiaux), la position du centre de la tête fémorale C2.5, visible à la figure 3, comme étant une approximation par la méthode des moindres carrés du centre de rotation autour duquel tourne l'ensemble tibial de capteurs inertiels 12 lorsque l'ensemble tibial de capteurs inertiels 12 suit le premier mouvement circulaire 32, représenté à la figure 14.

À partir des signaux générés par les gyromètres 4 et accéléromètres 6 (fémoraux) et par les gyromètres 14 et accéléromètres 16 (tibiaux), l'unité de calcul 30 est configurée pour calculer la position du centre du genou C3 comme étant une approximation par la méthode des moindres carrés du centre de rotation autour duquel tourne l'ensemble tibial de capteurs inertiels 12, lorsque l'ensemble tibial de capteurs inertiels 12 suit le deuxième mouvement circulaire composé d'une flexion de la jambe 36 supérieure à 90 degrés et d'une extension inverse 38.

L'unité de calcul 30 est en outre configurée pour déterminer la position d'un axe mécanique fémoral X20 et la position d'un axe mécanique tibial X23 qui sont représentés à la figure 3. L'unité de calcul 30 est en outre configurée pour calculer un angle cuisse-jambe s'étendant, du côté médial, entre l'axe mécanique fémoral X20 et l'axe mécanique tibial X23.

L'unité de calcul est configurée pour calculer, à partir des signaux générés par les gyromètres 4 et accéléromètres 6 (fémoraux) et par les gyromètres 14 et accéléromètres 16 (tibiaux) :
i) un axe médiolatéral tibial Y23 correspondant à la direction passant par la malléole médiale M26.1 et par la malléole latérale M26.2 ; et
ii) un axe médiolatéral fémoral Y20 porté par un vecteur moyen de rotation du référentiel XYZ tibial par rapport au référentiel XYZ fémoral, sur une plage angulaire de flexion s'étendant de 40 degrés à 80 degrés.

Le dispositif d'évaluation comprend en outre une mémoire 35 incluant un modèle anatomique du membre inférieur M1. Le modèle anatomique inclue : i) le centre de la tête fémorale C2.5, ii) le centre du genou C3, iii) le centre de la cheville C7, iv) l'axe mécanique fémoral X20 passant par le centre de la tête fémorale C2.5 et par le centre du genou C3, et v) l'axe mécanique tibial X23 passant par le centre du genou C3 et par le centre de la cheville C7.

L'ensemble fémoral de capteurs inertiels 2 comprend en outre une interface fémorale de commande tactile 62. De même, l'ensemble tibial de capteurs inertiels 12 comprend en outre une interface tibiale de commande tactile 63. L'interface fémorale de commande tactile 62 et l'interface tibiale de commande tactile 63 permettent au praticien de transmettre des instructions aux ensembles fémoral 2 et tibial 12 de capteurs inertiels et, le cas échéant, de visualiser des informations envoyées par les ensembles fémoral 2 et tibial 12 de capteurs inertiels 2.

Le dispositif d'évaluation 1 comprend en outre une interface de commande 64 qui est configurée pour permettre au praticien de commander l'unité de calcul 30. L'interface de commande 64 comprend un module d'affichage 66. De plus, le dispositif d'évaluation 1 comprend un module de communication sans fil 68 qui est configuré pour communiquer les résultats des calculs précités à l'interface de commande 64.

La figure 18 illustre un procédé d'évaluation 100 pour évaluer paramètres morphologiques du membre inférieur M1, en particulier pendant une consultation préopératoire ou postopératoire. Le procédé d'évaluation 100 comprend les étapes :
- 102) Mettre en oeuvre le dispositif d'évaluation 1.
- 104) Lorsque le dispositif d'évaluation 1 est en service, le dos du patient repose sur une table de consultation horizontale. Le praticien met en marche le dispositif d'évaluation 1, de façon à alimenter électriquement les ensembles fémoral 2 et tibial 12 de capteurs inertiels.
- 106) Le praticien manipule la cuisse M20 du patient en passant ses doigts sous la partie distale 41.2 de la barre fémorale 41, de façon à placer le membre inférieur en hyperextension. Ensuite, le praticien manipule le membre inférieur M1, par exemple en tenant le pied M29, pour induire le premier mouvement circulaire 32. Pendant le premier mouvement circulaire 32, symbolisé à la figure 14, le bassin du patient est immobile. L'ensemble fémoral de capteurs inertiels 2 et l'ensemble tibial de capteurs inertiels 12 suivent le premier mouvement circulaire 32, car le membre inférieur M1 est verrouillé en hyperextension.
Les gyromètres fémoraux 4, les accéléromètres fémoraux 6, les gyromètres tibiaux 14 et les accéléromètres tibiaux 16 génèrent des signaux représentatifs de leurs vitesses angulaires et de leurs accélérations linéaires respectives par rapport aux trois dimensions X, Y, Z du référentiel galiléen.
L'unité de calcul 30 reçoit ces signaux. Puis, l'unité de calcul 30 calcule la position du centre de la tête fémorale C2.5 à partir de ces signaux reçus.
- 108) Le praticien immobilise la cuisse M20 en position d'extension et il manipule la jambe M23 de sorte que la jambe M23 suit un deuxième mouvement circulaire, symbolisé à la figure 15, qui est composé d'une flexion 36 d'environ 110 degrés, puis d'une extension 38 pour ramener la jambe M23 en position d'extension.
Les gyromètres tibiaux 14 et les accéléromètres tibiaux 16 génèrent des signaux représentatifs de leurs vitesses angulaires et de leurs accélérations linéaires respectives par rapport aux trois dimensions X, Y, Z du référentiel galiléen.
L'unité de calcul 30 reçoit ces signaux. Puis, l'unité de calcul 30 calcule la position du centre du genou C3 à partir de ces signaux reçus.
- 110) Le praticien repose la jambe M23 en position d'extension. Initialement, les branches médiale 26.1 et latérale 26.2 sont en configuration distante, sans contact avec les malléoles médiale M26.1 et latérale M26.2.
   - Lorsque le membre inférieur M1 est en extension ou hyperextension et que le pied M29 est immobile, suite à une instruction entrée par le praticien sur l'interface de commande 64 ou sur l'interface fémorale 62 ou tibiale 63 de commande tactile, l'unité de calcul 30 calcule la position de la jambe M23 ou du tibia M33, puis cette position est mémorisée dans la mémoire 35. À cet instant, le support fémoral 23 et l'ensemble tibial de capteurs inertiels 12 sont immobiles, car ils sont maintenus par les sangles tibiales proximale 24.1 et distale 24.2.
   - Puis, le praticien déplace la branche médiale 26.1 et latérale 26.2 vers la malléole médiale M26.1, puis la branche latérale 26.2 vers la malléole latérale M26.2, jusqu'à la configuration de contact symbolisée par les flèches aux figures 16 et 17.
   - Le praticien entre, sur l'interface de commande 64 ou sur l'interface fémorale 62 ou tibiale 63 de commande tactile, une instruction de prise de mesure par l'ensemble tibial de capteurs inertiels 12.
   - Puis, en maintenant la jambe M23, donc le tibia M33, immobile, la sangle tibiale distale 24.2 est détendue, ce qui décolle un peu le support 23 du tibia M33.
   - Ensuite, la branche médiale 26.1 est mise en contact de la malléole médiale M26.1 en déplaçant par pivotement la portion proximale de fixation tibiale 24. À cet instant, les gyromètres tibiaux 14 et les accéléromètres tibiaux 16 génèrent des signaux représentatifs de leurs vitesses angulaires et de leurs accélérations linéaires respectives par rapport aux trois dimensions X, Y, Z du référentiel galiléen. À partir de ces signaux, l'unité de calcul 30 calcule la position atteinte par la branche médiale 26.1, position qui est enregistrée dans la mémoire 35.
   - De même, la branche latérale 26.2 est mise en contact de la malléole latérale M26.2 en déplaçant par pivotement la portion proximale de fixation tibiale 24. À partir des signaux générés par les gyromètres tibiaux 14 et les accéléromètres tibiaux 16, l'unité de calcul 30 calcule la position atteinte par la branche latérale 26.2, position qui est enregistrée dans la mémoire 35.
   - Le support tibial 23 et l'ensemble tibial de capteurs inertiels 12 sont ramenés en position initiale sur la jambe M23 en extension.
   - L'unité de calcul 30 calcule les déplacements par pivotements médial et latéral et détermine l'axe mécanique tibial X23 comme étant le milieu des deux déplacements par pivotements médial et latéral.
   - Puis, l'unité de calcul 30 calcule la position du centre de la cheville C7, comme étant le milieu 26.7 du segment 26.6 délimité par les zones de contact des branches médiale 26.1 et latérale 26.2.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet.

## Revendications

1. Dispositif d'évaluation (1), pour évaluer des paramètres morphologiques d'un membre inférieur (M1) d'un patient, en particulier pendant une consultation préopératoire ou postopératoire, le dispositif d'évaluation (1) étant **caractérisé en ce qu'**il comprend au moins :
- un ensemble fémoral de capteurs inertiels (2) comprenant trois gyromètres fémoraux (4) et trois accéléromètres fémoraux (6), les gyromètres fémoraux (4) étant configurés pour générer respectivement des signaux représentatifs de leurs vitesses angulaires respectives autour des trois dimensions (X, Y, Z) d'un référentiel galiléen, les accéléromètres fémoraux (6) étant configurés pour générer des signaux représentatifs de leurs accélérations linéaires respectives selon les trois dimensions (X, Y, Z) du référentiel galiléen,
- un ensemble tibial de capteurs inertiels (12) comprenant trois gyromètres tibiaux (14) et trois accéléromètres tibiaux (16), les gyromètres tibiaux (14) étant configurés pour générer respectivement des signaux représentatifs de leurs vitesses angulaires respectives autour des trois dimensions (X, Y, Z) d'un référentiel galiléen, les accéléromètres tibiaux (16) étant configurés pour générer des signaux représentatifs de leurs accélérations linéaires respectives selon les trois dimensions (X, Y, Z) du référentiel galiléen,
- un support fémoral (20) configuré pour maintenir l'ensemble fémoral de capteurs inertiels (2) sur la cuisse (M20) du patient, le support fémoral (20) ayant : i) une portion proximale de fixation fémorale (21) configurée pour fixer le support fémoral (20) sur une portion proximale (M21) de la cuisse (M20), et ii) une portion distale de fixation fémorale (22) configurée pour fixer le support fémoral (20) sur une portion distale (M22) de la cuisse (M20),
- un support tibial (23) configuré pour maintenir l'ensemble tibial de capteurs inertiels (12) sur la jambe (M23) du patient, le support tibial (23) ayant : i) une portion proximale de fixation tibiale (24) configurée pour fixer le support tibial (23) sur une portion proximale de la jambe (M24) et ii) une portion distale de fixation tibiale (25) configurée pour fixer le support tibial (23) sur une portion distale (M25) de la jambe (26),
- une unité de calcul (30) configurée pour recevoir les signaux générés par les gyromètres fémoraux (4), par les accéléromètres fémoraux (6), par les gyromètres tibiaux (14) et par les accéléromètres tibiaux (16), où l'unité de calcul (30) est configurée pour :
• recevoir lesdits signaux générés lorsque l'ensemble tibial de capteurs inertiels (12) suit un premier mouvement circulaire (32) induit lorsque le membre inférieur (M1) en extension ou en hyperextension tourne autour de la tête fémorale (M2.5) du patient,
• calculer, à partir des signaux reçus, la position du centre de la tête fémorale (C2.5),
• recevoir lesdits signaux générés lorsque l'ensemble fémoral de capteurs inertiels (2) est immobile, la cuisse (M20) du patient étant en immobile et en extension ou en hyperextension, et lorsque l'ensemble tibial de capteurs inertiels (12) suit un deuxième mouvement circulaire induit par au moins une flexion (36) de la jambe (M23) supérieure à 60 degrés, par exemple supérieure à 90 degrés,,
• calculer, à partir des signaux reçus, la position du centre du genou (C3),
ledit dispositif d'évaluation (1) étant **caractérisé en ce qu'**il comprend en outre un palpeur malléolaire (26) fixé au support tibial (23), le palpeur malléolaire (26) comprenant une branche médiale (26.1) et une branche latérale (26.2) qui sont articulées au support tibial (23) de façon à être déplaçables entre :
i) une configuration de contact, dans laquelle une zone de contact de la branche médiale (26.1) et une zone de contact de la branche latérale (26.2) sont mises en contact respectivement avec la malléole médiale (M26.1) et avec la malléole latérale (M26.2), et
ii) une configuration distante, dans laquelle la branche médiale (26.1) et la branche latérale (26.2) sont distantes respectivement de la malléole médiale (M26.1) et de la malléole latérale (M26.2), et
et **en ce que** l'unité de calcul (30) est en outre configurée pour :
• recevoir lesdits signaux générés lorsque la branche médiale (26.1) et la branche latérale (26.2) sont mises en contact respectivement avec la malléole médiale (M26.1) et avec la malléole latérale (M26.2) pendant que le pied (M29) est immobile, et
• calculer, à partir desdits signaux reçus, la position du centre de la cheville (C7) comme étant le milieu du segment délimité par la zone de contact de la branche médiale (26.1) et la zone de contact de la branche latérale (26.2) lorsque la branche médiale (26.1) et la branche latérale (26.2) sont en configuration de contact.

2. Dispositif d'évaluation (1) selon la revendication précédente, dans lequel le support fémoral (20) comprend un élément fémoral de liaison (40) reliant mécaniquement la portion proximale de fixation fémorale (21) à la portion distale de fixation fémorale (22), l'élément fémoral de liaison (40) étant rigide, l'ensemble fémoral de capteurs inertiels (2) étant solidarisé à l'élément fémoral de liaison (40).

3. Dispositif d'évaluation (1) selon la revendication précédente, dans lequel l'élément fémoral de liaison (40) comprend une barre fémorale (41) s'étendant de la portion proximale de fixation fémorale (21) à la portion distale de fixation fémorale (22), la barre fémorale (41) ayant : i) une partie proximale (41.1) rectiligne et destinée à s'étendre le long de la cuisse (M20), et ii) une partie distale (41.2) curviligne et configurée pour le passage de plusieurs doigts entre la cuisse (M20) et la partie distale (41.2).

4. Dispositif d'évaluation (1) selon l'une quelconque des revendications précédentes, dans lequel le support tibial (23) comprend un élément tibial de liaison (42) reliant mécaniquement la portion proximale de fixation tibiale (24) à la portion distale de fixation tibiale (25), l'élément tibial de liaison (42) étant rigide, l'ensemble tibial de capteurs inertiels (12) étant solidarisé à l'élément tibial de liaison (42).

5. Dispositif d'évaluation (1) selon la revendication précédente, dans lequel l'élément tibial de liaison (42) comprend une barre tibiale (43) s'étendant de la portion proximale de fixation tibiale (24) à la portion distale de fixation tibiale (25), la barre tibiale (43) ayant : i) une partie proximale (43.1) rectiligne et destinée à s'étendre le long de la cuisse (M20), et ii) une partie distale (43.2) curviligne et configurée pour le passage de plusieurs doigts entre la jambe (M23) et la partie distale (43.2).

6. Dispositif d'évaluation (1) selon l'une quelconque des revendications précédentes, dans lequel la portion proximale de fixation fémorale (21) a une surface proximale de contact (46) qui est incurvée et destinée à s'appuyer contre une surface proximale de la cuisse (M20.46), et dans lequel la portion distale de fixation fémorale (22) a une surface distale de contact (48) qui est incurvée et destinée à s'appuyer contre une surface distale de la cuisse (M20.48).

7. Dispositif d'évaluation (1) selon l'une quelconque des revendications précédentes, dans lequel le support tibial (23) comprend une partie tibiale télescopique (52) agencée entre la portion proximale de fixation tibiale (24) et la portion distale de fixation tibiale (25), la partie tibiale télescopique (52) étant configurée pour régler la longueur (L23) du support tibial (23).

8. Dispositif d'évaluation (1) selon l'une quelconque des revendications précédentes, dans lequel la portion proximale de fixation fémorale (21) comprend au moins une sangle fémorale proximale (21.1) qui est configurée pour entourer la portion proximale de la cuisse (M20), et dans lequel la portion distale de fixation fémorale (22) comprend au moins une sangle fémorale distale (21.2) qui est configurée pour entourer la portion distale (M22) de la cuisse (M20),
et dans lequel la portion proximale de fixation tibiale (24) comprend au moins une sangle tibiale proximale (24.1) qui est configurée pour entourer la portion proximale (M24) de la jambe (M23), et dans lequel la portion distale de fixation tibiale (25) comprend au moins une sangle tibiale distale (24.2) qui est configurée pour entourer la portion distale (M25) de la jambe (M23),
la portion proximale de fixation tibiale (24) comprenant en outre : i) un organe de serrage proximal (24.5) configuré pour serrer simultanément les deux brins de la sangle tibiale proximale (24.1), et ii) un organe de serrage distal (24.6) configuré pour serrer simultanément les deux brins de la sangle tibiale distale (24.2).

9. Dispositif d'évaluation (1) selon l'une quelconque des revendications précédentes, comprenant en outre une liaison filaire (60) reliant électriquement l'ensemble fémoral de capteurs inertiels (2) et l'ensemble tibial de capteurs inertiels (12).

10. Dispositif d'évaluation (1) selon la revendication précédente, dans lequel l'ensemble fémoral de capteurs inertiels (2) est équipé d'une batterie (61) configurée pour alimenter électriquement l'ensemble fémoral de capteurs inertiels (2) et l'ensemble tibial de capteurs inertiels (12).

11. Dispositif d'évaluation (1) selon l'une quelconque des revendications précédentes, dans lequel l'unité de calcul (30) est configurée pour calculer, à partir des signaux reçus, la position du centre de la tête fémorale (C2.5) comme étant une approximation par la méthode des moindres carrés du centre de rotation autour duquel tourne l'ensemble tibial de capteurs inertiels (12) lorsque l'ensemble tibial de capteurs inertiels (12) suit le premier mouvement circulaire (32).

12. Dispositif d'évaluation (1) selon l'une quelconque des revendications précédentes, dans lequel l'unité de calcul (30) est configurée pour calculer, à partir des signaux reçus, la position du centre du genou (C3) comme étant une approximation par la méthode des moindres carrés du centre de rotation autour duquel tourne l'ensemble tibial de capteurs inertiels (12) lorsque l'ensemble tibial de capteurs inertiels (12) suit le deuxième mouvement circulaire (36, 38).

13. Dispositif d'évaluation (1) selon l'une quelconque des revendications précédentes, dans lequel l'unité de calcul (30) est en outre configurée pour déterminer la position d'un axe mécanique fémoral (X20) et la position d'un axe mécanique tibial (X23), dans lequel l'unité de calcul (30) est en outre configurée pour calculer un angle cuisse-jambe s'étendant, du côté médial, entre l'axe mécanique fémoral (X20) et l'axe mécanique tibial (X23).

14. Dispositif d'évaluation (1) selon l'une quelconque des revendications précédentes, dans lequel l'ensemble fémoral de capteurs inertiels (2) comprend en outre une interface fémorale de commande tactile (62), et dans lequel l'ensemble tibial de capteurs inertiels (12) comprend en outre une interface tibiale de commande tactile (63).

15. Dispositif d'évaluation (1) selon l'une quelconque des revendications précédentes, comprenant en outre :
- une interface de commande (64) configurée pour permettre à un utilisateur de commander l'unité de calcul (30), l'interface de commande (64) comprenant un module d'affichage (66), et
- au moins un module de communication sans fil (68) configuré pour communiquer les résultats desdits calculs à l'interface de commande (64).

16. Procédé d'évaluation (100) pour évaluer des paramètres morphologiques d'un membre inférieur (M1) d'un patient, en particulier pendant une consultation préopératoire ou postopératoire, ledit procédé d'évaluation (100) mettant en oeuvre les étapes suivantes :
- mettre en oeuvre un dispositif d'évaluation (1) selon l'une quelconque des revendications précédentes, sur ledit membre inférieur (M1) ;
- lorsque le dispositif d'évaluation (1) est en service, mettre en marche le dispositif d'évaluation (1) de façon à alimenter électriquement l'ensemble fémoral de capteurs inertiels (2) et l'ensemble tibial de capteurs inertiels (12) ;
- manipuler le membre inférieur (M1) pour que l'unité de calcul (30) reçoive les signaux générés par les gyromètres fémoraux (4), par les accéléromètres fémoraux (6), par les gyromètres tibiaux (14) et par les accéléromètres tibiaux (16), de sorte que l'unité de calcul (30) :
• reçoive lesdits signaux générés lorsque l'ensemble tibial de capteurs inertiels (12) suit un premier mouvement circulaire (32) induit lorsque le membre inférieur (M1) en extension ou en hyperextension tourne autour de la tête fémorale (M2.5) du patient,
• calcule, à partir des signaux reçus, la position du centre de la tête fémorale (C2.5),
• reçoive lesdits signaux générés lorsque l'ensemble fémoral de capteurs inertiels (2) est immobile, la cuisse (M20) du patient étant en immobile et en extension ou en hyperextension, et lorsque l'ensemble tibial de capteurs inertiels (12) suit un deuxième mouvement circulaire induit par au moins une flexion (36) de la jambe (M23) supérieure à 60 degrés, par exemple supérieure à 90 degrés,,
• calcule, à partir des signaux reçus, la position du centre du genou (C3),
• reçoive lesdits signaux générés lorsque la branche médiale (26.1) et la branche latérale (26.2) sont mises en contact respectivement avec la malléole médiale (M26.1) et avec la malléole latérale (M26.2) pendant que le pied (M29) est immobile, et
• calcule, à partir desdits signaux reçus, la position du centre de la cheville (C7) comme étant le milieu du segment délimité par la zone de contact de la branche médiale (26.1) et la zone de contact de la branche latérale (26.2) lorsque la branche médiale (26.1) et la branche latérale (26.2) sont en configuration de contact.

## Patentansprüche

1. Bewertungsvorrichtung (1) zum Bewerten morphologischer Parameter eines unteren Gliedmaßes (M1) eines Patienten, insbesondere während einer präoperativen oder postoperativen Konsultation, wobei die Bewertungsvorrichtung (1) **dadurch gekennzeichnet ist, dass** sie mindestens Folgendes umfasst:
- eine Femoralträgheitssensoreneinheit (2), die drei Femoralgyrometer (4) und drei Femoralbeschleunigungsmesser (6) umfasst, wobei die Femoralgyrometer (4) konfiguriert sind, um jeweils Signale zu erzeugen, die für ihre jeweiligen Winkelgeschwindigkeiten um die drei Dimensionen (X, Y, Z) eines Inertialsystems repräsentativ sind, wobei die Femoralbeschleunigungsmesser (6) konfiguriert sind, um Signale zu erzeugen, die für ihre jeweiligen linearen Beschleunigungen entlang der drei Dimensionen (X, Y, Z) des Inertialsystems repräsentativ sind,
- eine Tibiaträgheitssensoreneinheit (12), die drei Tibiagyrometer (14) und drei Tibiabeschleunigungsmesser (16) umfasst, wobei die Tibiagyrometer (14) konfiguriert sind, um jeweils Signale zu erzeugen, die für ihre jeweiligen Winkelgeschwindigkeiten um die drei Dimensionen (X, Y, Z) eines Inertialsystems repräsentativ sind, wobei die Tibiabeschleunigungsmesser (16) konfiguriert sind, um Signale zu erzeugen, die für ihre jeweiligen linearen Beschleunigungen entlang der drei Dimensionen (X, Y, Z) des Inertialsystems repräsentativ sind,
- einen Femoralträger (20), der konfiguriert ist, um die Femoralträgheitssensoreneinheit (2) auf dem Oberschenkel (M20) des Patienten zu halten, wobei der Femoralträger (20) Folgendes aufweist: i) einen proximalen Femoralbefestigungsabschnitt (21), der konfiguriert ist, um den Femoralträger (20) auf einem proximalen Abschnitt (M21) des Oberschenkels (M20) zu befestigen, und ii) einen distalen Femoralbefestigungsabschnitt (22), der konfiguriert ist, um den Femoralträger (20) auf einem distalen Abschnitt (M22) des Oberschenkels (M20) zu befestigen,
- einen Tibiaträger (23), der konfiguriert ist, um die Tibiaträgheitssensoreneinheit (12) auf dem Bein (M23) des Patienten zu halten, wobei der Tibiaträger (23) Folgendes aufweist: i) einen proximalen Tibiabefestigungsabschnitt (24), der konfiguriert ist, um den Tibiaträger (23) auf einem proximalen Abschnitt des Beins (M24) zu befestigen, und ii) einen distalen Tibiabefestigungsabschnitt (25), der konfiguriert ist, um den Tibiaträger (23) auf einem distalen Abschnitt (M25) des Beins (26) zu befestigen,
- eine Recheneinheit (30), die konfiguriert ist, um die Signale zu empfangen, die von den Femoralgyrometern (4), von den Femoralbeschleunigungsmessern (6), von den Tibiagyrometern (14) und von den Tibiabeschleunigungsmessern (16) erzeugt werden, wobei die Recheneinheit (30) konfiguriert ist, um:
• die erzeugten Signale zu empfangen, wenn die Tibiaträgheitssensoreneinheit (12) einer ersten kreisförmigen Bewegung (32) folgt, die induziert wird, wenn das untere Gliedmaß (M1) in Extension oder in Hyperextension um den Oberschenkelknochenkopf (M2.5) des Patienten dreht,
• ausgehend von den empfangenen Signalen die Position der Mitte des Oberschenkelknochenkopfs (C2.5) zu berechnen,
• die erzeugten Signale zu empfangen, wenn die Femoralträgheitssensoreneinheit (2) immobil ist, wobei der Oberschenkel (M20) des Patienten immobil und in Extension oder in Hyperextension ist, und wenn die Tibiaträgheitssensoreneinheit (12) einer zweiten kreisförmigen Bewegung folgt, die durch mindestens eine Biegung (36) des Beins (M23) größer als 60 Grad, zum Beispiel größer als 90 Grad, induziert wird,
• ausgehend von den empfangenen Signalen die Position der Mitte des Knies (C3) zu berechnen,
wobei die Bewertungsvorrichtung (1) **dadurch gekennzeichnet ist, dass** sie außerdem einen Knöchelfühler (26) umfasst, der an dem Tibiaträger (23) befestigt ist, wobei der Knöchelfühler (26) einen medialen Zweig (26.1) und einen lateralen Zweig (26.2) umfasst, die an dem Tibiaträger (23) auf verschiebbare Art befestigt sind zwischen:
i) einer Kontaktkonfiguration, in der eine Kontaktzone des medialen Zweigs (26.1) und eine Kontaktzone des lateralen Zweigs (26.2) jeweils mit dem medialen Knöchel (M26.1) und dem lateralen Knöchel (M26.2) in Kontakt gebracht werden, und
ii) einer beabstandete Konfiguration, in der der mediale Zweig (26.1) und der laterale Zweig (26.2) jeweils von dem medialen Knöchel (M26.1) und dem lateralen Knöchel (M26.2) entfernt sind, und
und dadurch, dass die Recheneinheit (30) außerdem konfiguriert ist, um:
• die erzeugten Signale zu empfangen, wenn der mediale Zweig (26.1) und der laterale Zweig (26.2) jeweils mit dem medialen Knöchel (M26.1) und dem lateralen Knöchel (M26.2) in Kontakt gebracht werden, während der Fuß (M29) immobil ist, und
• ausgehend von den empfangenen Signalen die Position der Mitte des Fußgelenks (C7) als die Mitte des Segments zu berechnen, das von der Kontaktzone des medialen Zweigs (26.1) und der Kontaktzone des lateralen Zweigs (26.2) abgegrenzt ist, wenn der mediale Zweig (26.1) und der laterale Zweig (26.2) in Kontaktkonfiguration sind.

2. Bewertungsvorrichtung (1) nach dem vorstehenden Anspruch, wobei der Femoralträger (20) ein Femoralverbindungselement (40) umfasst, das den proximalen Femoralbefestigungsabschnitt (21) mechanisch mit dem distalen Femoralbefestigungsabschnitt (22) verbindet, wobei das Femoralverbindungselement (40) starr ist, wobei die Femoralträgheitssensoreneinheit (2) fest mit dem Femoralverbindungselement (40) verbunden ist.

3. Bewertungsvorrichtung (1) nach dem vorstehenden Anspruch, wobei das Femoralverbindungselement (40) eine Femoralstange (41) umfasst, die sich von dem proximalen Femoralbefestigungsabschnitt (21) zu dem distalen Femoralbefestigungsabschnitt (22) erstreckt, wobei die Femoralstange (41) Folgendes aufweist: i) einen proximalen Teil (41.1), der geradlinig und dazu bestimmt ist, sich entlang des Oberschenkels (M20) zu erstrecken, und einen ii) distalen Teil (41.2), der kurvilinear und für das Durchgehen mehrerer Finger zwischen dem Oberschenkel (M20) und dem distalen Teil (41.2) konfiguriert ist.

4. Bewertungsvorrichtung (1) nach einem der vorstehenden Ansprüche, wobei der Tibiaträger (23) ein Tibiaverbindungselement (42) umfasst, das den proximalen Tibiabefestigungsabschnitt (24) mechanisch mit dem distalen Tibiabefestigungsabschnitt (25) verbindet, wobei das Tibiaverbindungselement (42) starr ist, wobei die Tibiaträgheitssensoreneinheit (12) fest mit dem Tibiaverbindungselement (42) verbunden ist.

5. Bewertungsvorrichtung (1) nach dem vorstehenden Anspruch, wobei das Tibiaverbindungselement (42) eine Tibiastange (43) umfasst, die sich von dem proximalen Tibiabefestigungsabschnitt (24) zu dem distalen Tibiabefestigungsabschnitt (25) erstreckt, wobei die Tibiastange (43) Folgendes aufweist: i) einen proximalen Teil (43.1), der geradlinig und dazu bestimmt ist, sich entlang des Oberschenkels (M20) zu erstrecken, und einen ii) distalen Teil (43.2), der kurvilinear und für das Durchgehen mehrerer Finger zwischen dem Bein (M23) und dem distalen Teil (43.2) konfiguriert ist.

6. Bewertungsvorrichtung (1) nach einem der vorstehenden Ansprüche, wobei der proximale Femoralbefestigungsabschnitt (21) eine proximale Kontaktoberfläche (46) aufweist, die gebogen und dazu bestimmt ist, sich gegen eine proximale Oberfläche des Oberschenkels (M20.46) zu stützen, und wobei der distale Femoralbefestigungsabschnitt (22) eine distale Kontaktoberfläche (48) aufweist, die gebogen und dazu bestimmt ist, sich gegen eine distale Oberfläche des Oberschenkels (M20.48) zu stützen.

7. Bewertungsvorrichtung (1) nach einem der vorstehenden Ansprüche, wobei der Tibiaträger (23) einen teleskopischen Tibiateil (52) umfasst, der zwischen dem proximalen Tibiabefestigungsabschnitt (24) und dem distalen Tibiabefestigungsabschnitt (25) eingerichtet ist, wobei der teleskopische Tibiateil (52) konfiguriert ist, um die Länge (L23) des Tibiaträgers (23) zu regeln.

8. Bewertungsvorrichtung (1) nach einem der vorstehenden Ansprüche, wobei der proximale Femoralbefestigungsabschnitt (21) mindestens einen proximalen Femoralgurt (21.1) umfasst, der konfiguriert ist, um den proximalen Abschnitt des Oberschenkels (M20) zu umgeben, und wobei der distale Femoralbefestigungsabschnitt (22) mindestens einen distalen Femoralgurt (21.2) umfasst, der konfiguriert ist, um den distalen Abschnitt (M22) des Oberschenkels (M20) zu umgeben,
und wobei der proximale Tibiabefestigungsabschnitt (24) mindestens einen proximalen Tibiagurt (24.1) umfasst, der konfiguriert ist, um den proximalen Abschnitt (M24) des Beins (M23) zu umgeben, und wobei der distale Tibiabefestigungsabschnitt (25) mindestens einen distalen Tibiagurt (24.2) umfasst, der konfiguriert ist, um den distalen Abschnitt (M25) des Beins (M23) zu umgeben,
wobei der proximale Tibiabefestigungsabschnitt (24) außerdem Folgendes umfasst: i) ein proximales Spannorgan (24.5), das konfiguriert ist, um gleichzeitig die zwei Stränge des proximalen Tibiagurts (24.1) zu spannen, und ii) ein distales Spannorgan (24.6), das konfiguriert ist, um gleichzeitig die zwei Stränge des distalen Tibiagurts (24.2) zu spannen.

9. Bewertungsvorrichtung (1) nach einem der vorstehenden Ansprüche, die außerdem eine Drahtverbindung (60) umfasst, die die Femoralträgheitssensoreneinheit (2) und die Tibiaträgheitssensoreneinheit (12) elektrisch verbindet.

10. Bewertungsvorrichtung (1) nach dem vorstehenden Anspruch, wobei die Femoralträgheitssensoreneinheit (2) mit einer Batterie (61) ausgestattet ist, die konfiguriert ist, um die Femoralträgheitssensoreneinheit (2) und die Tibiaträgheitssensoreneinheit (12) elektrisch zu versorgen.

11. Bewertungsvorrichtung (1) nach einem der vorstehenden Ansprüche, wobei die Recheneinheit (30) konfiguriert ist, um ausgehend von den empfangenen Signalen die Position der Mitte des Oberschenkelknochenkopfs (C2.5) als eine Annäherung durch das Verfahren der kleinsten Quadrate der Rotationsmitte zu berechnen, um die die Tibiaträgheitssensoreneinheit (12) dreht, wenn die Tibiaträgheitssensoreneinheit (12) der ersten kreisförmigen Bewegung (32) folgt.

12. Bewertungsvorrichtung (1) nach einem der vorstehenden Ansprüche, wobei die Recheneinheit (30) konfiguriert ist, um ausgehend von den empfangenen Signalen die Position der Mitte des Knies (C3) als eine Annäherung durch das Verfahren der kleinsten Quadrate der Rotationsmitte zu berechnen, um die die Tibiaträgheitssensoreneinheit (12) dreht, wenn die Tibiaträgheitssensoreneinheit (12) der zweiten kreisförmigen Bewegung (36, 38) folgt.

13. Bewertungsvorrichtung (1) nach einem der vorstehenden Ansprüche, wobei die Recheneinheit (30) außerdem konfiguriert ist, um die Position einer mechanischen Femoralachse (X20) und die Position einer mechanischen Tibiaachse (X23) zu bestimmen, wobei die Recheneinheit (30) außerdem konfiguriert ist, um einen Winkel zwischen Oberschenkel und Bein, der sich auf der medialen Seite zwischen der mechanischen Femoralachse (X20) und der mechanischen Tibiaachse (X23) erstreckt, zu berechnen.

14. Bewertungsvorrichtung (1) nach einem der vorstehenden Ansprüche, wobei die Femoralträgheitssensoreneinheit (2) außerdem eine taktile Femoralsteuerschnittstelle (62) umfasst, und wobei die Tibiaträgheitssensoreneinheit (12) außerdem eine taktile Tibiasteuerschnittstelle (63) umfasst.

15. Bewertungsvorrichtung (1) nach einem der vorstehenden Ansprüche, die außerdem Folgendes umfasst:
- eine Steuerschnittstelle (64), die konfiguriert ist, um es einem Benutzer zu erlauben, die Recheneinheit (30) zu steuern, wobei die Steuerschnittstelle (64) ein Anzeigemodul (66) umfasst, und
- mindestens ein drahtloses Kommunikationsmodul (68), das konfiguriert ist, um die Resultate der Berechnungen zu der Steuerschnittstelle (64) zu kommunizieren.

16. Bewertungsverfahren (100) zum Bewerten morphologischer Parameter eines unteren Gliedmaßes (M1) eines Patienten, insbesondere während einer präoperativen oder postoperativen Konsultation, wobei das Bewertungsverfahren (100) die folgenden Schritte umsetzt:
- Umsetzen einer Bewertungsvorrichtung (1) nach einem der vorstehenden Ansprüche auf dem unteren Gliedmaß (M1);
- wenn die Bewertungsvorrichtung (1) in Betrieb ist, Einschalten der Bewertungsvorrichtung (1) derart, dass die Femoralträgheitssensoreneinheit (2) und die Tibiaträgheitssensoreneinheit (12) elektrisch versorgt werden;
- Manipulieren des unteren Gliedmaßes (M1), damit die Recheneinheit (30) die Signale empfängt, die von den Femoralgyrometern (4), den Femoralbeschleunigungsmessern (6), den Tibiagyrometern (14) und den Tibiabeschleunigungsmessern (16) erzeugt werden, so dass die Recheneinheit (30):
• die erzeugten Signale empfängt, wenn die Tibiaträgheitssensoreneinheit (12) einer ersten kreisförmigen Bewegung (32) folgt, die induziert wird, wenn das untere Gliedmaß (M1) in Extension oder in Hyperextension um den Oberschenkelknochenkopf (M2.5) des Patienten dreht,
• ausgehend von den empfangenen Signalen die Position der Mitte des Oberschenkelknochenkopfs (C2.5) berechnet,
• die erzeugten Signale empfängt, wenn die Femoralträgheitssensoreneinheit (2) immobil ist, wobei der Oberschenkel (M20) des Patienten immobil und in Extension oder in Hyperextension ist, und wenn die Tibiaträgheitssensoreneinheit (12) einer zweiten kreisförmigen Bewegung folgt, die durch mindestens eine Biegung (36) des Beins (M23) größer als 60 Grad, zum Beispiel größer als 90 Grad, induziert wird,
• ausgehend von den empfangenen Signalen die Position der Mitte des Knies (C3) berechnet,
• die erzeugten Signale empfängt, wenn der mediale Zweig (26.1) und der laterale Zweig (26.2) jeweils mit dem medialen Knöchel (M26.1) und mit dem lateralen Knöchel (M26.2) in Kontakt gebracht werden, während der Fuß (M29) immobil ist, und
• ausgehend von den empfangenen Signalen die Position der Mitte des Fußgelenks (C7) als die Mitte des Segments berechnet, das von der Kontaktzone des medialen Zweigs (26.1) und der Kontaktzone des lateralen Zweigs (26.2) abgegrenzt wird, wenn der mediale Zweig (26.1) und der laterale Zweig (26.2) in Kontaktkonfiguration sind.

## Claims

1. An assessment device (1), for assessing morphological parameters of a lower limb (M1) of a patient, in particular during a preoperative or postoperative consultation, the assessment device (1) being **characterized in that** it comprises at least:
- a femoral set of inertial sensors (2) comprising three femoral gyrometers (4) and three femoral accelerometers (6), the femoral gyrometers (4) being configured to generate respectively signals representative of their respective angular velocities about the three dimensions (X, Y, Z) of a Galilean frame of reference, the femoral accelerometers (6) being configured to generate signals representative of their respective linear accelerations according to the three dimensions (X, Y, Z) of the Galilean frame of reference,
- a tibial set of inertial sensors (12) comprising three tibial gyrometers (14) and three tibial accelerometers (16), the tibial gyrometers (14) being configured to generate respectively signals representative of their respective angular velocities about the three dimensions (X, Y, Z) of a Galilean frame of reference, the tibial accelerometers (16) being configured to generate signals representative of their respective linear accelerations according to the three dimensions (X, Y, Z) of the Galilean frame of reference,
- a femoral support (20) configured to hold the femoral set of inertial sensors (2) on the thigh (M20) of the patient, the femoral support (20) having: i) a proximal femoral fastening portion (21) configured to fasten the femoral support (20) on a proximal portion (M21) of the thigh (M20), and ii) a distal femoral fastening portion (22) configured to fasten the femoral support (20) on a distal portion (M22) of the thigh (M20),
- a tibial support (23) configured to hold the tibial set of inertial sensors (12) on the leg (M23) of the patient, the tibial support (23) having: i) a proximal tibial fastening portion (24) configured to fasten the tibial support (23) on a proximal portion of the leg (M24) and ii) a distal tibial fastening portion (25) configured to fasten the tibial support (23) on a distal portion (M25) of the leg (26),
- a calculation unit (30) configured to receive the signals generated by the femoral gyrometers (4), by the femoral accelerometers (6), by the tibial gyrometers (14) and by the tibial accelerometers (16), where the calculation unit (30) is configured to:
• receive said generated signals when the tibial set of inertial sensors (12) follows a first circular movement (32) induced when the lower limb (M1) in extension or in hyperextension rotates around the femoral head (M2.5) of the patient,
• calculate, from the received signals, the position of the center of the femoral head (C2.5),
• receive said generated signals when the femoral set of inertial sensors (2) is stationary, the thigh (M20) of the patient being stationary and in extension or in hyperextension, and when the tibial set of inertial sensors (12) follows a second circular movement induced by at least one flexion (36) of the leg (M23) larger than 60 degrees, for example larger than 90 degrees,
• calculate, from the received signals, the position of the center of the knee (C3),
said assessment device (1) being **characterized in that** it further comprises a malleolar palpating probe (26) fastened to the tibial support (23), the malleolar palpating probe (26) comprising a medial branch (26.1) and a lateral branch (26.2) which are hinged to the tibial support (23) so as to be displaceable between:
i) a contact configuration, in which a contact area of the medial branch (26.1) and a contact area of the lateral branch (26.2) are contacted respectively with the medial malleolus (M26.1) and with the lateral malleolus (M26.2), and
ii) a distant configuration, in which the medial branch (26.1) and the lateral branch (26.2) are distant respectively from the medial malleolus (M26.1) and the lateral malleolus (M26.2), and
**in that** the calculation unit (30) is further configured to:
• receive said generated signals when the medial branch (26.1) and the lateral branch (26.2) are contacted respectively with the medial malleolus (M26.1) and with the lateral malleolus (M26.2) while the foot (M29) is motionless, and
• calculate, from said received signals, the position of the center of the ankle (C7) as the middle of the segment delimited by the contact area of the medial branch (26.1) and the contact area of the lateral branch (26.2) when the medial branch (26.1) and the lateral branch (26.2) are in contact configuration.

2. The assessment device (1) according to the preceding claim, wherein the femoral support (20) comprises a femoral connecting element (40) mechanically connecting the proximal femoral fastening portion (21) to the distal femoral fastening portion (22), the femoral connecting element (40) being rigid, the femoral set of inertial sensors (2) being secured to the femoral connecting element (40).

3. The assessment device (1) according to the preceding claim, wherein the femoral connecting element (40) comprises a femoral bar (41) extending from the proximal femoral fastening portion (21) to the distal femoral fastening portion (22), the femoral bar (41) having: i) a rectilinear proximal portion (41.1) intended to extend along the thigh (M20), and ii) a curvilinear distal portion (41.2) configured for the passage of several fingers between the thigh (M20) and the distal portion (41.2).

4. The assessment device (1) according to any one of the preceding claims, wherein the tibial support (23) comprises a tibial connecting element (42) mechanically connecting the proximal tibial fastening portion (24) to the distal tibial fastening portion (25), the tibial connecting element (42) being rigid, the tibial set of inertial sensors (12) being secured to the tibial connecting element (42).

5. The assessment device (1) according to the preceding claim, wherein the tibial connecting element (42) comprises a tibial bar (43) extending from the proximal tibial fastening portion (24) to the distal tibial fastening portion (25), the tibial bar (43) having: i) a rectilinear proximal portion (43.1) intended to extend along the thigh (M20), and ii) a curvilinear distal portion (432) configured for the passage of several fingers between the leg (M23) and the distal portion (43.2).

6. The assessment device (1) according to any one of the preceding claims, wherein the proximal femoral fastening portion (21) has a proximal contact surface (46) which is curved and intended to bear against a proximal surface of the thigh (M20.46), and wherein the distal femoral fastening portion (22) has a distal contact surface (48) which is curved and intended to bear against a distal surface of the thigh (M20.48).

7. The assessment device (1) according to any one of the preceding claims, wherein the tibial support (23) comprises a telescopic tibial portion (52) arranged between the proximal tibial fastening portion (24) and the distal tibial fastening portion (25), the telescopic tibial portion (52) being configured to adjust the length (L23) of the tibial support (23).

8. The assessment device (1) according to any one of the preceding claims, wherein the proximal femoral fastening portion (21) comprises at least one proximal femoral strap (21.1) which is configured to surround the proximal portion of the thigh (M20), and wherein the distal femoral fastening portion (22) comprises at least one distal femoral strap (21.2) which is configured to surround the distal portion (M22) of the thigh (M20),
and wherein the proximal tibial fastening portion (24) comprises at least one proximal tibial strap (24.1) which is configured to surround the proximal portion (M24) of the leg (M23), and wherein the distal tibial fastening portion (25) comprises at least one distal tibial strap (24.2) which is configured to surround the distal portion (M25) of the leg (M23),
the proximal tibial fastening portion (24) further comprising: i) a proximal clamping member (24.5) configured to simultaneously clamp the two strands of the proximal tibial strap (24.1), and ii) a distal clamping member (24.6) configured to simultaneously clamp the two strands of the distal tibial strap (24.2).

9. The assessment device (1) according to any one of the preceding claims, further comprising a wire connection (60) electrically connecting the femoral set of inertial sensors (2) and the tibial set of inertial sensors (12).

10. The assessment device (1) according to the preceding claim, wherein the femoral set of inertial sensors (2) is equipped with a battery (61) configured to supply electric power to the femoral set of inertial sensors (2) and the tibial set of inertial sensors (12).

11. The assessment device (1) according to any one of the preceding claims, wherein the calculation unit (30) is configured to calculate, from the received signals, the position of the center of the femoral head (C2.5) as an approximation by least squares method of the center of rotation about which the tibial set of inertial sensors (12) rotates when the tibial set of inertial sensors (12) follows the first circular movement (32).

12. The assessment device (1) according to any one of the preceding claims, wherein the calculation unit (30) is configured to calculate, from the received signals, the position of the center of the knee (C3) as an approximation by least squares method of the center of rotation about which the tibial set of inertial sensors (12) rotates when the tibial set of inertial sensors (12) follows the second circular movement (36, 38).

13. The assessment device (1) according to any one of the preceding claims, wherein the calculation unit (30) is further configured to determine the position of a femoral mechanical axis (X20) and the position of a tibial mechanical axis (X23), wherein the calculation unit (30) is further configured to calculate a thigh-leg angle extending, on the medial side, between the femoral mechanical axis (X20) and the tibial mechanical axis (X23).

14. The assessment device (1) according to any one of the preceding claims, wherein the femoral set of inertial sensors (2) further comprises a femoral touch control interface (62), and wherein the tibial set of inertial sensors (12) further comprises a tibial touch control interface (63).

15. The assessment device (1) according to any one of the preceding claims, further comprising:
- a control interface (64) configured to enable a user to control the calculation unit (30), the control interface (64) comprising a display module (66), and
- at least one wireless communication module (68) configured to communicate the results of said calculations to the control interface (64).

16. An assessment method (100) for assessing morphological parameters of a lower limb (M1) of a patient, in particular during a preoperative or postoperative consultation, said assessment method (100) implementing the following steps of:
- implementing an assessment device (1) according to any one of the preceding claims, on said lower limb (M1),
- when the assessment device (1) is operating, turning on the assessment device (1) so as to supply electric power to the femoral set of inertial sensors (2) and the tibial set of inertial sensors (12);
- manipulating the lower limb (M1) so that the calculation unit (30) receives the signals generated by the femoral gyrometers (4), by the femoral accelerometers (6), by the tibial gyrometers (14) and by the tibial accelerometers (16), so that the calculation unit (30):
• receives said generated signals when the tibial set of inertial sensors (12) follows a first circular movement (32) induced when the lower limb (M1) in extension or in hyperextension rotates around the femoral head (M2.5) of the patient,
• calculates, from the received signals, the position of the center of the femoral head (C2.5),
• receives said generated signals when the femoral set of inertial sensors (2) is stationary, the thigh (M20) of the patient being stationary and in extension or in hyperextension, and when the tibial set of inertial sensors (12) follows a second circular movement induced by at least one flexion (36) of the leg (M23) larger than 60 degrees, for example larger than 90 degrees,
• calculates, from the received signals, the position of the center of the knee (C3),
• receives said generated signals when the medial branch (26.1) and the lateral branch (26.2) are contacted respectively with the medial malleolus (M26.1) and with the lateral malleolus (M26.2) while the foot (M29) is stationary, and
• calculates, from said received signals, the position of the center of the ankle (C7) as the middle of the segment delimited by the contact area of the medial branch (26.1) and the contact area of the lateral branch (26.2) when the medial branch (26.1) and the lateral branch (26.2) are in contact configuration.
